(19) Europäisches Patentamt / European Patent Office / Office européen des brevets

(11) **EP 3 745 952 B1**

(12) **EUROPEAN PATENT SPECIFICATION**

(45) Date of publication and mention
of the grant of the patent:
**06.03.2024 Bulletin 2024/10**

(21) Application number: **19788241.8**

(22) Date of filing: **17.04.2019**

(51) International Patent Classification (IPC):
*A61B 5/021* (2006.01)   *A61B 5/06* (2006.01)
*A61B 5/00* (2006.01)   *A61B 5/024* (2006.01)

(52) Cooperative Patent Classification (CPC):
**A61B 5/02108; A61B 5/02438; A61B 5/065;
A61B 5/684; A61B 5/6844; A61B 5/7475;**
A61B 5/02416; A61B 5/681; A61B 5/6898;
A61B 2560/0223

(86) International application number:
**PCT/KR2019/004605**

(87) International publication number:
**WO 2019/203554 (24.10.2019 Gazette 2019/43)**

(54) **ELECTRONIC DEVICE AND METHOD OF CONTROLLING ELECTRONIC DEVICE**

ELEKTRONISCHE VORRICHTUNG UND VERFAHREN ZUR STEUERUNG EINER
ELEKTRONISCHEN VORRICHTUNG

DISPOSITIF ÉLECTRONIQUE ET PROCÉDÉ DE COMMANDE DE DISPOSITIF ÉLECTRONIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.04.2018 KR 20180044412**

(43) Date of publication of application:
**09.12.2020 Bulletin 2020/50**

(73) Proprietor: **Samsung Electronics Co., Ltd.
Gyeonggi-do 16677 (KR)**

(72) Inventors:
• **OH, Junseok**
  **Suwon-si**
  **Gyeonggi-do 16677 (KR)**
• **KIM, Jinho**
  **Suwon-si**
  **Gyeonggi-do 16677 (KR)**
• **SHIN, Seunghwan**
  **Suwon-si**
  **Gyeonggi-do 16677 (KR)**
• **YUN, Inho**
  **Suwon-si**
  **Gyeonggi-do 16677 (KR)**
• **CHO, Sunghwan**
  **Suwon-si**
  **Gyeonggi-do 16677 (KR)**

(74) Representative: **Arnold & Siedsma
Bezuidenhoutseweg 57
2594 AC The Hague (NL)**

(56) References cited:
EP-A1- 1 405 592    WO-A1-2014/094245
WO-A1-2017/111564    US-A1- 2010 125 212
US-A1- 2011 172 547    US-A1- 2011 237 963
US-A1- 2013 237 865    US-A1- 2014 218 496
US-A1- 2015 182 147    US-A1- 2016 310 071
US-A1- 2017 273 620    US-A1- 2017 281 044
US-A1- 2017 331 505    US-A1- 2017 332 963

EP 3 745 952 B1

**Description**

Technical Field

The disclosure relates to an electronic device for providing a function of guiding a position (POSE) for blood pressure (BP) measurement and a method of controlling an electronic device.

Background Art

Due to an increase in interest in health, electronic devices including biometric sensors have been developed. The electronic devices may provide information related to human health by acquiring information on states of human bodies.

Tomohiro Izumi et al. ("Wrist sphygmomanometer", US2011/0172547 A1) discloses a wrist sphygmomanometer including an operation unit operable by a user. A manometer measures blood pressure. A detector detects the posture of the user. A storage module stores an optimum posture for the user. A comparator compares the posture detected by the detector and the optimum posture stored beforehand in the storage to generate posture information.

Yi Liu et al. ("Sphygmomanometer system and blood pressure measurement method thereof, WO2014/094245 A1) discloses a sphygmomanometer system and a blood pressure measurement method thereof. The sphygmomanometer system is divided into a blood pressure measurement device and a mobile terminal.

The above information is presented as background information only to assist with an understanding of the disclosure. No determination has been made, and no assertion is made, as to whether any of the above might be applicable as prior art with regard to the disclosure.

Disclosure of Invention

Solution to Problem

An electronic device may estimate blood pressure (BP) by acquiring information related to a person's heart rate through one or more sensors. When information such as the heart rate is acquired, the information may vary depending on a position of a user holding or wearing the electronic device, for example, a height of the electronic device and a distance between the electronic device and the user.

Aspects of the disclosure are to address at least the above-mentioned problems and/or disadvantages and to provide at least the advantages described below. Accordingly, an aspect of the disclosure is to provide an electronic device and a method for guiding acquisition of information related to a heart rate at a predetermined position of the user all the time.

The technical subjects pursued in the disclosure may not be limited to the above mentioned technical subjects, and other technical subjects which are not mentioned may be clearly understood, through the following descriptions, by those skilled in the art of the disclosure.

Additional aspects will be set forth in part in the description which follows and, in part, will be apparent from the description, or may be learned by practice of the presented embodiments.

In accordance with an aspect of the present invention, an electronic device according to appended independent claim 1 is provided.

In accordance with another aspect of the present invention, a method according to appended independent claim 8 is provided.

In accordance with another aspect of the present invention, a computer program product according to appended independent claim 13 is provided.

Other aspects, advantages, and salient features of the disclosure will become apparent to those skilled in the art from the following detailed description, which, taken in conjunction with the annexed drawings, discloses various embodiments of the disclosure.

Brief Description of Drawings

The above and other aspects, features, and advantages of certain embodiments of the disclosure will be more apparent from the following description taken in conjunction with the accompanying drawings, in which:

FIG. 1 illustrates the form of an electronic device according to an embodiment of the disclosure;
FIG. 2 illustrates the form of the electronic device according to an embodiment of the disclosure;
FIG. 3 is a block diagram illustrating the electronic device within a network environment according to an embodiment of the disclosure;

FIGS. 4A and 4B illustrate positions of a user measuring biometric information through the electronic device according to various embodiments of the disclosure;

FIGS. 5A, 5B, and 5C illustrate a situation in which the electronic device acquires first data related to a first position of the user measuring biometric information of the user through the electronic device according to various embodiments of the disclosure;

FIGS. 6A and 6B illustrate a situation in which the electronic device acquires first data related to a first position of the user measuring user's biometric information according to various embodiments of the disclosure;

FIGS. 7A, 7B, and 7C illustrate a situation in which the electronic device provides a guide to measurement of biometric information to the user according to various embodiments of the disclosure;

FIGS 8A and 8B illustrate a situation in which the electronic device provides a guide to measure biometric information of the user according to various embodiments of the disclosure;

FIG. 9 is a block diagram schematically illustrating the electronic device according to an embodiment of the disclosure;

FIG. 10 is a flowchart illustrating a situation in which the electronic device acquires data for measuring biometric information of the user, identifies a position of the user, and provides a guide according to an embodiment of the disclosure;

FIG. 11 is a flowchart illustrating a situation in which the electronic device acquires a first position of the user according to an embodiment of the disclosure;

FIG. 12 is a flowchart illustrating a situation in which the electronic device provides a guide to a measurement position to the user when blood pressure (BP) is measured through the electronic device according to an embodiment of the disclosure;

FIG. 13 is a flowchart illustrating another situation in which the electronic device provides a guide to a measurement position to the user when BP is measured through the electronic device according to various embodiments of the disclosure;

FIGS. 14A and 14B illustrate a situation in which the margin of error is generated when a reference position of BP measurement pre-stored in the electronic device does not match a user's position of BP measurement according to various embodiments of the disclosure;

FIGS. 15A and 15B illustrate a situation in which the margin of error is generated when a reference position of BP measurement pre-stored in the electronic device does not match a user's position of BP measurement according to various embodiments of the disclosure;

FIGS. 16A and 16B illustrate a situation in which the margin of error is generated when a reference position of BP measurement pre-stored in the electronic device does not match a user's position of BP measurement according to various embodiment of the disclosure.

Throughout the drawings, it should be noted that like reference numbers are used to depict the same or similar elements, features, and structures.

Best Mode for Carrying out the Invention

The following description with reference to the accompanying drawings is provided to assist in a comprehensive understanding of various embodiments of the disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope and spirit of the disclosure. In addition, descriptions of well-known functions and constructions may be omitted for clarity and conciseness.

The terms and words used in the following description and claims are not limited to the bibliographical meanings, but, are merely used by the inventor to enable a clear and consistent understanding of the disclosure. Accordingly, it should be apparent to those skilled in the art that the following description of various embodiments of the disclosure is provided for illustration purpose only and not for the purpose of limiting the disclosure as defined by the appended claims and their equivalents.

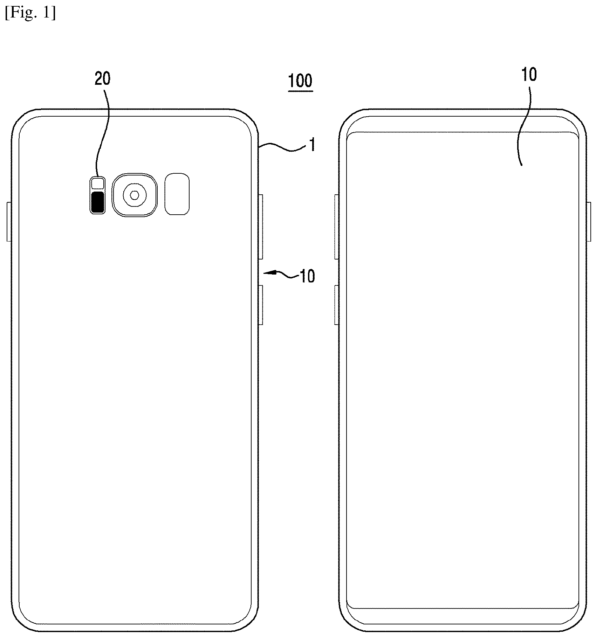

FIG. 1 illustrates the shape of an electronic device according to an embodiment of the disclosure.

Referring to FIG. 1, an electronic device 100 may include a housing 1, a display 10, and a biometric information-measuring optical sensor 20.

The housing 1 may provide a space for accommodating elements (for example, the display 10 and the biometric information-measuring optical sensor 20). The housing 1 may be implemented in various forms. Although FIG. 1 illustrates an example in which the housing 1 is implemented in a rectangular form having a curved surface to be held by a user, the housing 1 according to various embodiments may be implemented in various forms in which the user can hold the electronic device 100.

The display 10 may be used for providing information processed by the electronic device 100. According to various embodiments, the display 10 may display a screen or a user interface related to information processed by the electronic device 100. According to various embodiments, the display 10 may be disposed on a front surface of the housing 1 in order to provide information processed by the electronic device 100. According to various embodiments, the display 10 may be exposed through the part of the front surface of the housing 1 in order to provide information processed by the electronic device 100.

The biometric information-measuring optical sensor 20 may be used to measure biometric information of the user of the electronic device 100. The biometric information-measuring optical sensor 20 may be disposed on a rear surface or the front surface of the housing 1 in order to measure the pulse of the user of the electronic device 100. The rear surface may be a surface opposite the front surface. According to various embodiments, the biometric information-measuring optical sensor 20 may be exposed through the part of the rear surface or the front surface of the housing 1 in order to measure the pulse of the user of the electronic device 100. In FIG. 1, the surface on which the biometric information-measuring optical sensor 20 is disposed is the rear surface and the surface on which the display 10 is disposed is the front surface. However, the disclosure is not limited thereto.

The biometric information-measuring optical sensor 20 may be disposed on an upper portion of the rear surface of the housing 1 so that the user's body part (for example, fingers) can contact the biometric information-measuring optical sensor 20 while the user holds the electronic devices 100 (or the housing 1). However, the location of the biometric information-measuring optical sensor 20 is not limited thereto. The biometric information-measuring optical sensor 20 may be located at various positions of the electronic device 100 such that the part of the user's body contacts the biometric information-measuring optical sensor 20.

The electronic device 101 may further include a film (not shown) attached to, disposed on (over) or superimposed on (over), or overlaid on (over) the biometric information-measuring optical sensor 20. The film may be used to measure blood sugar and may include chemochromic materials. The film used to measure blood sugar may be configured to be translucent or transparent in order to apply light of a required wavelength to the user.

When the film is configured to be transparent, the biometric information-measuring optical sensor 20 may further include a device (for example, a light emitting diode) for emitting light of the required wavelength.

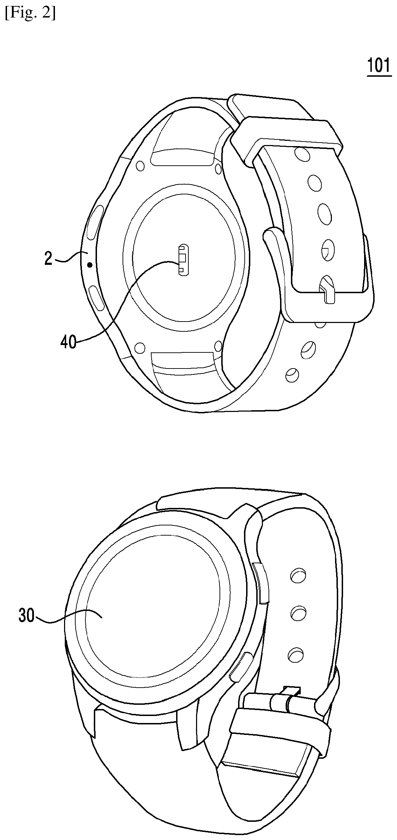

FIG. 2 illustrates the form of the electronic device according to an embodiment of the disclosure.

Referring to FIG. 2, the electronic device 101 may include a housing 2, a display 30, and a biometric information-measuring optical sensor 40.

The housing 2 may provide a space for accommodating elements (for example, the display 30 and the biometric information-measuring optical sensor 40). The housing 2 may be implemented in various forms. Although FIG. 2 illustrates an example in which the housing 2 is implemented in a circular form attachable to the user's body part, but is not limited thereto. According to various embodiments, the housing 2 may be implemented in another form other than the circle. According to various embodiments, the housing 2 may be implemented in a rectangle, a square, or an oval attachable to the user's body part.

The display 30 may be used for providing information processed by the electronic device 101. According to various embodiments, the display 30 may display a screen or a user interface related to information processed by the electronic device 101. According to various embodiments, the display 30 may be disposed on a front surface of the housing 2 in order to provide information processed by the electronic device 101. According to various embodiments, the display 30 may be exposed through the part of the front surface of the housing 2 in order to provide information processed by the electronic device 101.

The biometric information-measuring optical sensor 40 may be used to measure the pulse of the user of the electronic device 101. According to various embodiments, the biometric information-measuring optical sensor 40 may emit light toward the user's body part contacting the electronic device 101 through one or more light emitting diodes or lasers. The biometric information-measuring optical sensor 40 may receive reflected light of the emitted light through photodiodes or different types of light detectors. The biometric information-measuring optical sensor 40 may convert information on the reflected light into an electrical signal. The electrical signal acquired through the biometric information-measuring optical sensor 40 may be transferred to a processor within the electronic device 101. The transferred to electrical signal may include information on the user's pulse or pulse wave.

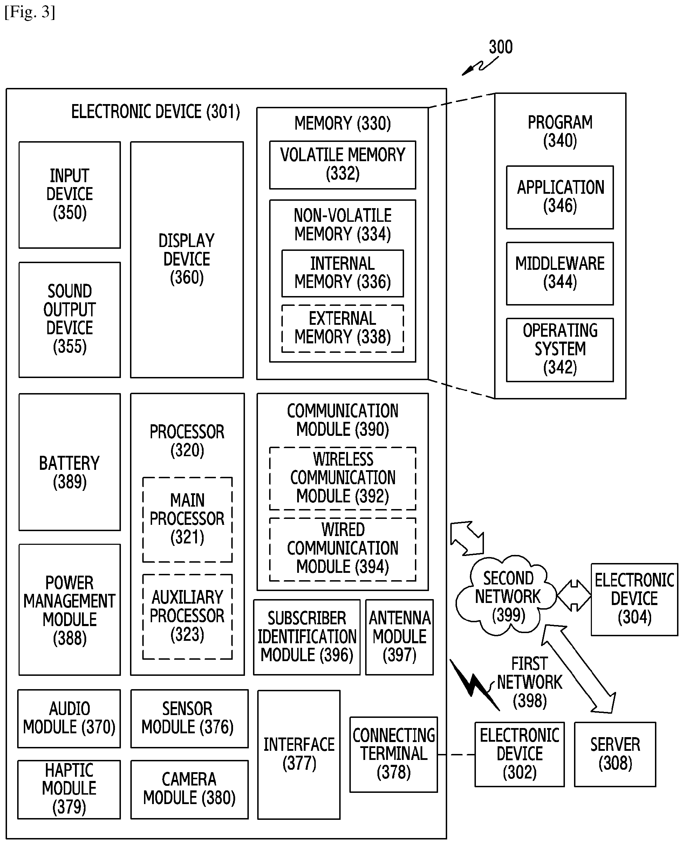

FIG. 3 is a block diagram illustrating an electronic device 301 in a network environment 300 according to an embodiment of the disclosure. An electronic device 301 may include the electronic device 100 of FIG. 1 and the electronic device 101 of FIG. 2.

Referring to FIG. 3, the electronic device 301 in the network environment 300 may communicate with an electronic device 302 via a first network 398 (e.g., a short-range wireless communication network), or an electronic device 304 or a server 308 via a second network 399 (e.g., a long-range wireless communication network). According to an embodiment, the electronic device 301 may communicate with the electronic device 304 via the server 308. According to an embodiment, the electronic device 301 may include a processor 320, memory 330, an input device 350, a sound output device 355, a display device 360, an audio module 370, a sensor module 376, an interface 377, a haptic module 379, a camera module 380, a power management module 388, a battery 389, a communication module 390, a subscriber identification module (SIM) 396, or an antenna module 397. In some embodiments, at least one (e.g., the display device 360 or the camera module 380) of the components may be omitted from the electronic device 301, or one or more other components may be added in the electronic device 301. In some embodiments, some of the components may be implemented as single integrated circuitry. For example, the sensor module 376 (e.g., a fingerprint sensor, an iris sensor, or an illuminance sensor) may be implemented as embedded in the display device 360 (e.g., a display).

The processor 320 may execute, for example, software (e.g., a program 340) to control at least one other component (e.g., a hardware or software component) of the electronic device 301 coupled with the processor 320, and may perform various data processing or computation. According to one embodiment, as at least part of the data processing or computation, the processor 320 may load a command or data received from another component (e.g., the sensor module 376 or the communication module 390) in volatile memory 332, process the command or the data stored in the volatile memory 332, and store resulting data in non-volatile memory 334. According to an embodiment, the processor 320 may include a main processor 321 (e.g., a central processing unit (CPU) or an application processor (AP)), and an auxiliary processor 323 (e.g., a graphics processing unit (GPU), an image signal processor (ISP), a sensor hub processor, or a communication processor (CP)) that is operable independently from, or in conjunction with, the main processor 321. Additionally or alternatively, the auxiliary processor 323 may be adapted to consume less power than the main processor 321, or to be specific to a specified function. The auxiliary processor 323 may be implemented as separate from, or as part of the main processor 321.

The auxiliary processor 323 may control at least some of functions or states related to at least one component (e.g., the display device 360, the sensor module 376, or the communication module 390) among the components of the electronic device 301, instead of the main processor 321 while the main processor 321 is in an inactive (e.g., sleep) state, or together with the main processor 321 while the main processor 321 is in an active state (e.g., executing an application). According to an embodiment, the auxiliary processor 323 (e.g., an ISP or a CP) may be implemented as part of another component (e.g., the camera module 380 or the communication module 390) functionally related to the auxiliary processor 323.

The memory 330 may store various data used by at least one component (e.g., the processor 320 or the sensor module 376) of the electronic device 301. The various data may include, for example, software (e.g., the program 340) and input data or output data for a command related thereto. The memory 330 may include the volatile memory 332 or the non-volatile memory 334.

The program 340 may be stored in the memory 330 as software, and may include, for example, an operating system (OS) 342, middleware 344, or an application 346.

The input device 350 may receive a command or data to be used by other component (e.g., the processor 320) of the electronic device 301, from the outside (e.g., a user) of the electronic device 301. The input device 350 may include, for example, a microphone, a mouse, a keyboard, or a digital pen (e.g., a stylus pen).

The sound output device 355 may output sound signals to the outside of the electronic device 301. The sound output device 355 may include, for example, a speaker or a receiver. The speaker may be used for general purposes, such as playing multimedia or playing record, and the receiver may be used for an incoming call. According to an embodiment, the receiver may be implemented as separate from, or as part of the speaker.

The display device 360 may visually provide information to the outside (e.g., a user) of the electronic device 301. The display device 360 may include, for example, a display, a hologram device, or a projector and control circuitry to control a corresponding one of the display, hologram device, and projector. According to an embodiment, the display device 360 may include touch circuitry adapted to detect a touch, or sensor circuitry (e.g., a pressure sensor) adapted to measure the intensity of force incurred by the touch.

The audio module 370 may convert a sound into an electrical signal and vice versa. According to an embodiment, the audio module 370 may obtain the sound via the input device 350, or output the sound via the sound output device 355 or a headphone of an external electronic device (e.g., an electronic device 302) directly (e.g., wired) or wirelessly coupled with the electronic device 301.

The sensor module 376 may detect an operational state (e.g., power or temperature) of the electronic device 301 or an environmental state (e.g., a state of a user) external to the electronic device 301, and then generate an electrical signal or data value corresponding to the detected state. According to an embodiment, the sensor module 376 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 377 may support one or more specified protocols to be used for the electronic device 301 to be coupled with the external electronic device (e.g., the electronic device 302) directly (e.g., wired) or wirelessly. According to an embodiment, the interface 377 may include, for example, a high definition multimedia interface (HDMI), a universal serial bus (USB) interface, a secure digital (SD) card interface, or an audio interface.

A connecting terminal 378 may include a connector via which the electronic device 301 may be physically connected with the external electronic device (e.g., the electronic device 302). According to an embodiment, the connecting terminal 378 may include, for example, a HDMI connector, a USB connector, a SD card connector, or an audio connector (e.g., a headphone connector).

The haptic module 379 may convert an electrical signal into a mechanical stimulus (e.g., a vibration or a movement) or electrical stimulus which may be recognized by a user via his tactile sensation or kinesthetic sensation. According to an embodiment, the haptic module 379 may include, for example, a motor, a piezoelectric element, or an electric stimulator.

The camera module 380 may capture a still image or moving images. According to an embodiment, the camera module 380 may include one or more lenses, image sensors, ISP, or flashes.

The power management module 388 may manage power supplied to the electronic device 301. According to one embodiment, the power management module 388 may be implemented as at least part of, for example, a power management integrated circuit (PMIC).

The battery 389 may supply power to at least one component of the electronic device 301. According to an embodiment, the battery 389 may include, for example, a primary cell which is not rechargeable, a secondary cell which is rechargeable, or a fuel cell.

The communication module 390 may support establishing a direct (e.g., wired) communication channel or a wireless communication channel between the electronic device 301 and the external electronic device (e.g., the electronic device 302, the electronic device 304, or the server 308) and performing communication via the established communication channel. The communication module 390 may include one or more CP that are operable independently from the processor 320 (e.g., the AP) and supports a direct (e.g., wired) communication or a wireless communication. According to an embodiment, the communication module 390 may include a wireless communication module 392 (e.g., a cellular communication module, a short-range wireless communication module, or a global navigation satellite system (GNSS) communication module) or a wired communication module 394 (e.g., a local area network (LAN) communication module or a power line communication (PLC) module). A corresponding one of these communication modules may communicate with the external electronic device via the first network 398 (e.g., a short-range communication network, such as Bluetooth™, Wi-Fi direct, or IR data association (IrDA)) or the second network 399 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 392 may identify and authenticate the electronic device 301 in a communication network, such as the first network 398 or the second network 399, using subscriber information (e.g., international mobile subscriber identity (IMSI)) stored in the subscriber identification module 396.

The antenna module 397 may transmit or receive a signal or power to or from the outside (e.g., the external electronic device) of the electronic device 301. According to an embodiment, the antenna module 397 may include an antenna including a radiating element composed of a conductive material or a conductive pattern formed in or on a substrate (e.g., a printed circuit board (PCB)). According to an embodiment, the antenna module 397 may include a plurality of antennas. In such a case, at least one antenna appropriate for a communication scheme used in the communication network, such as the first network 398 or the second network 399, may be selected, for example, by the communication module 390 (e.g., the wireless communication module 392) from the plurality of antennas. The signal or the power may then be transmitted or received between the communication module 390 and the external electronic device via the selected at least one antenna. According to an embodiment, another component (e.g., a radio frequency integrated circuit (RFIC)) other than the radiating element may be additionally formed as part of the antenna module 397.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 301 and the external electronic device 304 via the server 308 coupled with the second network 399. Each of the electronic devices 302 and 304 may be a device of a same type as, or a different type, from the electronic device 301. According to an embodiment, all or some of operations to be executed at the electronic device 301 may be executed at one or more of the external electronic devices 302, 304, or 308. For example, if the electronic device 301 should perform a function or a service automatically, or in response to a request from a user or another device, the electronic device 301, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 301. The electronic device 301 may provide the outcome, with or without further processing of the outcome, as at least part of a reply to the request. To that end, a cloud computing, distributed computing, or client-server computing technology may be used, for example.

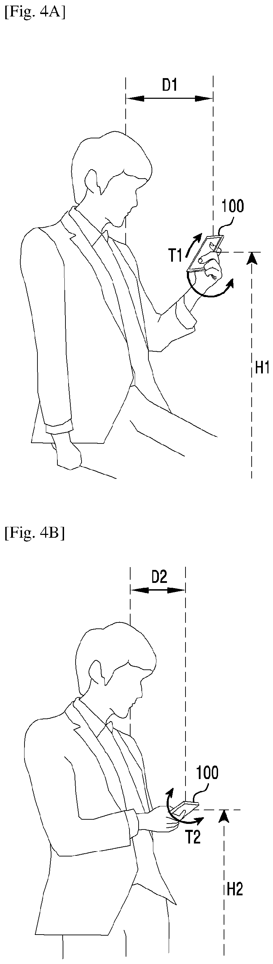

FIGS. 4A and 4B illustrate positions of the user measuring biometric information through the electronic device according to various embodiments of the disclosure.

Referring to FIGS. 4A and 4B, the electronic device 100 may acquire various pieces of biometric information through a biometric information-measuring optical sensor (the biometric information-measuring optical sensor 20 of FIG. 1) and measure blood pressure (BP). BP may be understood as, for example, pressure applied to walls of blood vessel by blood from the heart. The electronic device 100 according to an embodiment may measure BP through various methods.

The electronic device 100 may measure BP on the basis of, for example, a tonometry type, or the pulse wave such as a pulse wave velocity (PWV) type or a pulse wave analysis (PWA) type. The pulse wave may be understood as waves formed while the pulse is transmitted to peripheral nerves, that is, a change in pressure. The pulse is, for example, blood flow along the artery through heart beat and may be understood as repetition of expansion and relaxation of the artery.

The PWV type is a type of estimating BP on the basis of a time different of the pulse wave measured at two positions after pressure sensors are disposed at different locations of the blood vessel of the artery. For example, the PWV type may analyze a plurality of heartbeat-related signals (for example, pulse wave, ballistocardiogram, and electrocardiogram (ECG)), infer a blood flow rate, and estimate the BP through a correlation between the blood flow rate and the BP.

The PWA type is a type of estimating the BP on the basis of a shape of the pulse wave. For example, the PWA type is a type of inferring a change in the BP according to the shape of a waveform by analyzing the pulse wave on a terminal body or a wrist.

According to an embodiment, when the user measures BP through the electronic device 100 including the biometric information-measuring optical sensor 20, the electronic device 100 may provide a user interface including guide information for measuring the BP with a second position of the user which is the same as or similar to a pre-stored first position of the user in order to improve accuracy of the measured value.

According to an embodiment, the first position of the user may be a position which is a reference for measuring the BP. The position which is the reference for measuring the BP may be, for example, a position with which the user previously measured the BP through the electronic device 100 according to a reference position guide provided by the electronic device 100.

However, the disclosure is not limited thereto and the position which is the reference for measuring the BP may be a position pre-stored in the electronic device 100. For example, the position which is the reference for measuring the BP may be a state in which the user can see the display 10 of the electronic device 100 while raising the electronic device 100 to the same height as the user's chest. However, the position which is the reference is not limited thereto.

For example, if a request for measuring user's BP is made (again) after the BP is measured with the position which is the reference for measuring the BP, the electronic device 100 may display a user interface for guiding the user to take the second position in order to allow the user to measure the BP with the position which is the same as or similar to the first position of the user through the electronic device 100. For example, the electronic device 100 may acquire and store first data related to the first position of the user, and then may identify the second position of the user on the basis of second data measured through a sensor module included in the electronic device 100 and identify whether a difference between the first data and the second data is within a preset allowable range. For example, the second position may be a position identified using the second data acquired through the sensor module included in the electronic device 100 in the state in which the user is measuring the BP again after measuring the BP with the first position.

According to an embodiment, the first data and the second data related to the first position and the second position of the user may include at least one of a height of the electronic device 100, a distance between the electronic device 100 and the user, and an inclination of the electronic device 100.

Referring to FIG. 4A, the height (H1) of the electronic device 100 included in the first data may be an absolute height acquired through an altitude sensor or an air pressure sensor included in the electronic device 100. However, the height is not limited thereto. For example, the height (H1) of the electronic device 100 included in the first data may be a height from the lowest point in the distance between a point at which the standing user raises his/her arm holding or wearing the electronic device 100 over his/her head as much as possible and a point at which the user lowers his/her arm toward his/her legs.

According to various embodiments, the distance (D1) between the electronic device 100 and the user included in the first data may be a distance between the electronic device 100 and the user's chest in the state in which the user holds or wears the electronic device 100.

According to various embodiments, the inclination (T1) of the electronic device 100 included in the first data may be a tilting degree of the electronic device 100 against a horizontal plane in the state in which the user holds the electronic device 100.

Referring to FIG. 4B, the height (H2) of the electronic device 100 included in the second data may be an absolute height acquired through an altitude sensor or an air pressure sensor included in the electronic device 100. However, the height is not limited thereto. For example, the height (H2) of the electronic device 100 included in the second data may be a distance measured on the basis of the height (H1) of the electronic device included in the first data.

According to various embodiments, the distance (D2) between the electronic device 100 and the user included in the second data may be a distance between the electronic device 100 and the user's chest in the state in which the user holds or wears the electronic device 100. However, the distance is not limited thereto. For example, the distance (D2) between the electronic device 100 and the user included in the second data may be a distance measured on the basis of the distance (D1) between the electronic device 100 and the user included in the first data.

According to various embodiments, the inclination (T2) of the electronic device 100 included in the second data may be a tilting degree of the electronic device 100 against a horizontal plane in the state in which the user holds the electronic device 100. However, the inclination is not limited thereto. For example, the inclination of the electronic device 100 included in the second data may be an inclination measured on the basis of the inclination (T1) of the electronic device 100 included in the first data.

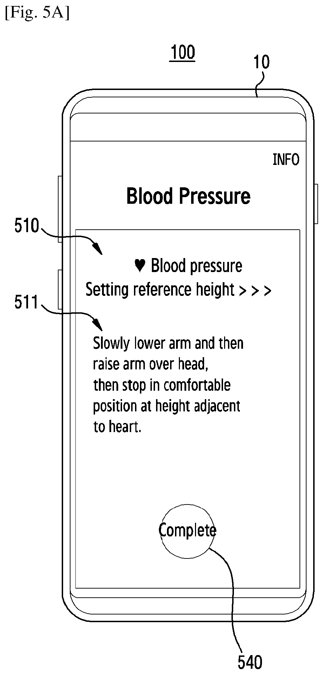

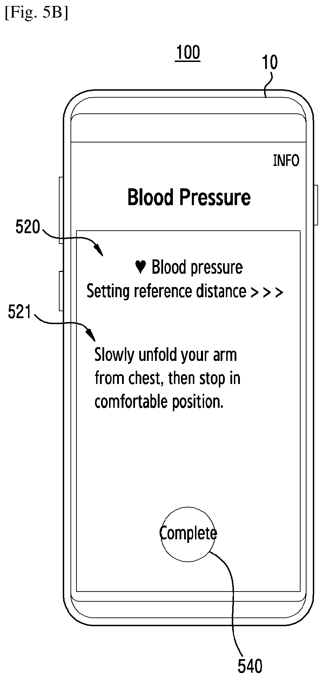

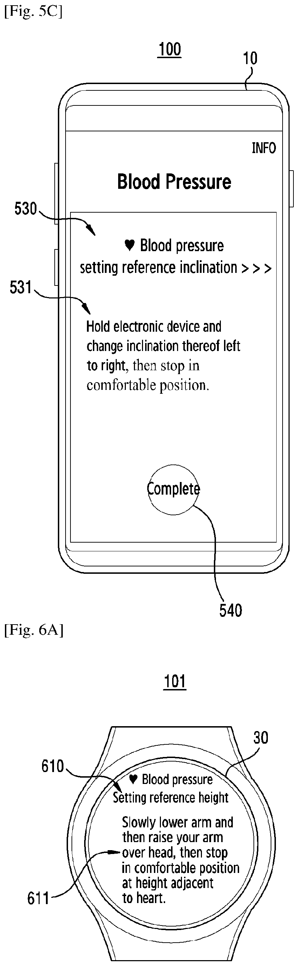

FIGS. 5A, 5B, and 5C illustrate a situation in which the electronic device acquires first data related to a first position of the user measuring biometric information of the user through the electronic device according to various embodiments of the disclosure.

Referring to FIGS. 5A to 5C, the electronic device 100 may display a user interface for acquiring first data related to the first position of the user. According to an embodiment, the electronic device 100 may provide a particular mode for acquiring the first data related to the first position of the user. For example, when a biometric information acquisition function is first performed by the electronic device 100, the electronic device 100 may display a user interface for storing a reference position of measuring the BP.

Referring to FIG. 5A, the electronic device 100 may display a reference height acquisition user interface 510 including guide information for acquiring a height of the electronic device 100 included in first data on the display 10.

The reference height acquisition user interface 510 may display information 511 making a request for lowering the arm holding the electronic device 100 toward legs, slowly raising the arm toward the head, and then stopping at the height adjacent to the user's heart to the user.

The electronic device 100 according to an embodiment may measure the height of the electronic device 100, a movement direction, or an amount of a change in the height through a sensor module included in the electronic device 100. The electronic device 100 may acquire first data related to a first position of the user on the basis of the data acquired through the sensor.

For example, the electronic device 100 may acquire, as first data, data input in the state in which the electronic device 100 moves according to the information 511 displayed on the display 10 on the basis of a value acquired through the sensor module and then stops at a predetermined location for a preset time (for example, 2 to 3 seconds).

According to some embodiments, the electronic device 100 may acquire, as first data, data input at the time point corresponding to user's selection of a "completion" icon 540 after the electronic device 100 moves according to the information 511 displayed on the display 10 on the basis of the value acquired through the sensor module.

According to an embodiment, when the first data is acquired, the electronic device 100 may inform the user that the first data has completely acquired through vibration, a sound, or a change in the user interface (for example, a change in color or brightness).

Referring to FIG. 5B, the electronic device 100 may display a reference distance acquisition user interface 520 including guide information for acquiring the distance between the electronic device 100 and the user included in the first data on the display 10.

The reference distance acquisition user interface 520 may display information 521 making a request for moving the electronic device 100 away from the user's chest and then stopping the electronic device in a comfortable position of the user to the user.

The electronic device 100 according to an embodiment may measure the distance between the electronic device 100 and the user through the sensor module included in the electronic device 100. The electronic device 100 may measure the location of the electronic device 100, the movement direction, or the movement distance. The electronic device 100 may acquire first data related to the first position of the user on the basis of the data acquired through the sensor.

For example, after moving according to the information 521 displayed on the display 10 on the basis of a value acquired through the sensor module, the electronic device 100 may acquire data input for a preset time (for example, 2 to 3 seconds) at a predetermined location in a stopped state as the first data.

According to some embodiments, after moving according to the information 521 displayed on the display 10 on the basis of the value acquired through the sensor module, the electronic device 100 may acquire data input at the time point corresponding to user's selection of the "completion" icon 540 as the first data.

According to an embodiment, when the first data is acquired, the electronic device 100 may inform the user that the first data has completely acquired through vibration, a sound, or a change in the user interface (for example, a change in color or brightness).

Referring to FIG. 5C, the electronic device 100 may display a reference inclination acquisition user interface 530 including guide information for acquiring the inclination of the electronic device 100 included in the first data on the display 10.

The reference inclination acquisition user interface 530 may display information 531 making a request for changing the inclination and then stopping in a comfortable position of the user to the user in the state in which the user holds the electronic device 100.

The electronic device 100 according to an embodiment may measure the inclination of the electronic device 100 through the sensor module included in the electronic device 100. The electronic device 100 may acquire first data related to the first position on the basis of data acquired through the sensor module.

For example, the electronic device 100 may acquire, as first data, data input in the state in which the electronic device 100 stops for a preset time (for example, 2 to 3 seconds) at a predetermined location after the inclination is changed according to the information 531 displayed on the display 10 and then on the basis of the value acquired through the sensor module.

According to various embodiments, the electronic device 100 may acquire, as first data, data input at the time point corresponding to user's selection of the "completion" icon 540 after the inclination of the electronic device 100 is changed according to the information 531 displayed on the display 10 on the basis of the value acquired through the sensor module.

According to an embodiment, when the first data is acquired, the electronic device 100 may inform the user that the first data has completely acquired through vibration, a sound, or a change in the user interface (for example, a change in color or brightness).

According to various embodiments, the electronic device 100 may simultaneously or sequentially display the user interface 510, 520, or 530 and output a sound or vibration corresponding to guide information. The electronic device 100 may output only a sound or vibration corresponding to guide information. For example, the electronic device 100 may output pieces of information 511, 521, and 531 displayed in the user interfaces 510, 520, and 530 through a voice or in various forms of vibration.

According to an embodiment, when the electronic device 100 acquires BP at the first position of the user according to the description of FIG. 5, the electronic device 100 may acquire accurate BP on the basis of BP measured using a cuff BP monitor.

When the BP is measured using the PWV type, the electronic device 100 according to an embodiment may measure the BP through Equation 1 and Equation 2.

$$BP = -\frac{2}{\alpha} \ln PTT + \frac{\ln \frac{2r \rho L^2}{hE_0}}{\alpha}$$

$$....\text{Equation } 1$$

In Equation 1, BP denotes BP measured using a cuff BP monitor at the first position, PTT denotes a pulse wave measured by the electronic device 100, r denotes a radius of a blood vessel, $\rho$ denotes blood viscosity, L denotes a length of a blood vessel, h denotes a width of a wall of a blood vessel, E denotes a coefficient of elasticity, and $\alpha$ denotes a corrected value. The electronic device 100 may acquire the corrected value $\alpha$ through the BP measured by, for example, the cuff BP monitor. Thereafter, the electronic device 100 may derive the BP on the basis of the acquired biometric information and corrected value $\alpha$. .

$$BP_n = a \cdot \ln PTT + b \cdot HR + c \cdot BP_{n-1} + d$$

$$....\text{Equation } 2$$

In Equation 2, HR denotes a heart rate.

The electronic device 100 according to an embodiment may calculate the corrected value $\alpha$ through Equation 1 above and acquire the next BPs $BP_n$ by applying the pre-measured BP $BP_{n-1}$ to Equation 2.

When the BP is measured using the PWA type, the electronic device 100 according to an embodiment may measure the BP through Equation 3 and Equation 4.

$$BP = BP_{baseline} + \Delta BP$$

….Equation 3

In Equation 3, BP denotes BP to be measured, $BP_{baseline}$ denotes BP measured by a cuff BP monitor at a first position, and $\Delta BP$ denotes a degree of a change in the BP $BP_{baseline}$.

$$\Delta BP = \rho \cdot (wCO \cdot fCO_{norm} \cdot wTPR \cdot fTPR_{norm})$$

….Equation 4

In Equation 4, $\rho$ denotes blood viscosity, CO denotes cardiac output, TPR denotes total peripheral resistance, w denotes specific gravity of each change, $fCO_{norm}$ denotes a relative change of cardiac output (CO) feature points, and $fTPR_{norm}$ denotes a relative change of total peripheral resistance (TPR) feature points. The expression "norm" is an abbreviation of "normalization" and may be a relative value.

According to an embodiment, after calculating the degree of the pressure change on the basis of the blood viscosity, the cardiac output, and the total peripheral resistance, the electronic device 100 may derive the BP using the BP ($BP_{baseline}$)measured by the cuff BP monitor.

FIG. 6A illustrates a situation in which the electronic device acquires first data related to a first position of the user measuring user's biometric information according to various embodiments of the disclosure.

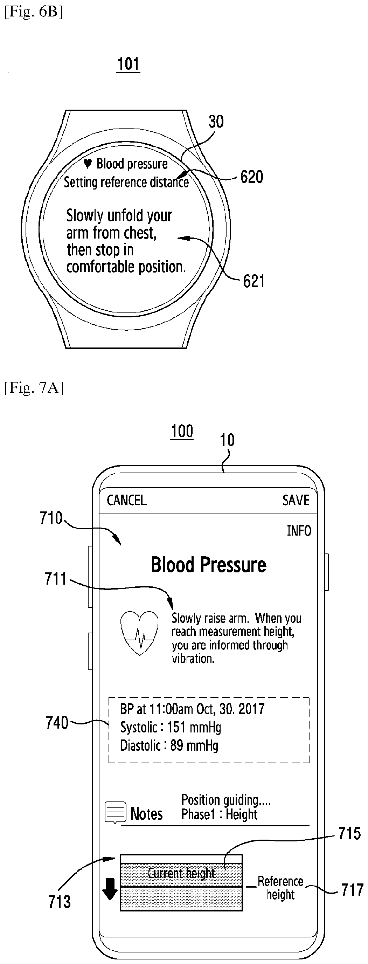

Referring to FIGS. 6A and 6B, the electronic device 101 may be a wearable device worn on the user's wrist. The electronic device 101 may display a user interface for acquiring first data related to the first position of the user.

Referring to FIG. 6A, the electronic device 101 may display a reference height acquisition user interface 610 including guide information for acquiring a height of the electronic device 101 included in first data on the display 30.

The reference height acquisition user interface 610 may display information 611 making, to the user, a request for lowering the arm holding the electronic device 101 toward legs, slowly raising the arm toward the head, and then stopping at the height adjacent to the user's heart.

The electronic device 101 according to an embodiment may measure the height of the electronic device 101, a movement direction, or an amount of a change in the height through a sensor module included in the electronic device 101. The electronic device 101 may acquire first data related to the first position on the basis of the data acquired through the sensor module.

For example, the electronic device 101 may acquire, as first data, data input in the state in which the electronic device 101 moves according to the information 611 displayed on the display 30 and then stops at a predetermined location for a preset time (for example, 2 to 3 seconds) on the basis of the value acquired through sensor module.

According to an embodiment, when the first data is acquired, the electronic device 101 may inform the user that the first data has completely acquired through vibration, a sound, or a change in the user interface (for example, a change in color or brightness).

Referring to FIG. 6B, the electronic device 101 may display the reference distance acquisition user interface 620 including guide information for acquiring a distance between the electronic device 101 and the user included in the first data on the display 30.

For example, the reference distance acquisition user interface 620 may display information 621 making a request for moving the electronic device 101 away from the user's chest and then stopping the electronic device 101 in a comfortable position of the user to the user.

The electronic device 101 according to an embodiment may measure the distance between the electronic device 101 and the user through the sensor module included in the electronic device 101. Further, the electronic device 101 may measure the location of the electronic device 101, the movement direction, or the movement distance. The electronic device 101 may acquire first data related to the first position of the user on the basis of the data acquired through the sensor module.

For example, the electronic device 101 may acquire, as first data, data input in the state in which the electronic device 101 moves according to the information 621 displayed on the display 30 on the basis of a value acquired through the sensor module and then stops at a predetermined location for a preset time (for example, 2 to 3 seconds).

According to an embodiment, when the first data is acquired, the electronic device 101 may inform the user that the first data has completely acquired through vibration, a sound, or a change in the user interface (for example, a change in color or brightness).

According to various embodiments, the electronic device 101 may sequentially display the user interface 610 or 620 and output a sound or vibration corresponding to guide information. The electronic device 101 may output only a sound or vibration corresponding to guide information. For example, the electronic device 101 may output pieces of information 611 and 621 displayed in the user interfaces 610 and 620 in various forms of vibration.

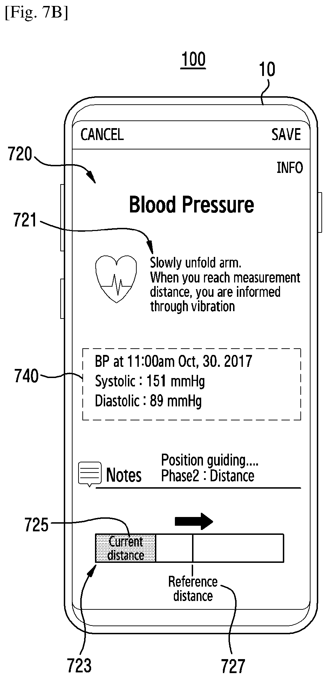

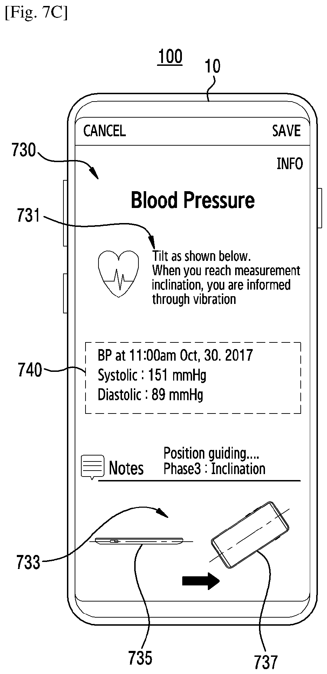

FIGS. 7A, 7B, and 7C illustrate a situation in which the electronic device provides a guide to measurement of biometric information to the user according to various embodiments of the disclosure.

Referring to FIGS. 7A, 7B, and 7C, the electronic device 100 may identify a second position of the user measuring BP on the basis of second data acquired using the sensor module included in the electronic device 100. The electronic device 100 may identify whether a difference between the first data related to the pre-stored first position of the user and the second data is included within a preset allowable range, and when the different is not within the allowable range, display a user interface including guide information for making the second position of the user become the same as the first position of the user.

According to an embodiment, when a user input for performing a function of acquiring biometric information is received, the electronic device 100 may perform a function of measuring biometric information (for example, BP, pulse, and oxygen saturation ($SPO_2$)). However, the disclosure is not limited thereto. For example, the electronic device 100 may perform the function of acquiring biometric information according to a preset period.

Referring to FIG. 7A, the electronic device 100 may identity whether the height of the electronic device 100 included in second data acquired through the sensor module and the height of the electronic device 100 included in pre-stored first data are included within a preset allowable range, and when the heights are not included within the allowable range, may display a height guide user interface 710 for making the second position of the user become the same as the first position of the user on the display 10.

The guide user interface 710 may display information 711 making a request for lowering the arm holding the electronic device 100 toward bottom and then slowly raising the arm toward the head to the user.

The electronic device 100 according to an embodiment may measure the height of the electronic device 100, the movement direction, or an amount of a change in the height through the sensor module included in the electronic device 100. For example, the electronic device 100 may measure the height of the electronic device 100 or the amount of the change in the height through an altitude sensor, an acceleration sensor, a gyro sensor, an air pressure sensor, a global positioning system (GPS) module, or a Wi-Fi module.

The electronic device 100 according to an embodiment may identify the second position of the user on the basis of the second data acquired through the sensor module. The electronic device 100 may display information related to the acquired second data and the pre-stored first data on the display 10.

Referring to FIG. 7A, the electronic device 100 may display the height of the electronic device 100 included in the first data and the height of the electronic device 100 included in the second data in the form of a graph 713. The electronic device 100 may display, for example, a reference height (for example, the height of the electronic device 100 included in the first data) and a current height (for example, the height of the electronic device 100 included in the second data) through the graph 713.

According to an embodiment, the electronic device 100 may reflect a value of the second data varying according to movement of the electronic device 100 to display the graph 713. For example, when a graph object 715 indicating the current height of the electronic device 100 is close or matches a graph object 717 indicating the reference height, the electronic device 100 may generate a notification. The electronic device 100 may make, for example, vibration, a sound, or a change (for example, a change in a color or brightness) in the screen of the height guide user interface 710 and output a notification.

Referring to FIG. 7B, the electronic device 100 may identify whether the distance between the electronic device 100 and the user included in the second data acquired through the sensor module and the distance between the electronic device 100 and the user included in the pre-stored first data are included within a preset allowable range, and when the distances are included within the allowable range, may display a distance guide user interface 720 for making the second position of the user become the same as the first position of the user on the display 10.

The distance guide user interface 720 may display, for example, information 721 making a request for moving the hand holding the electronic device 100 farther away from the chest to the user.

The electronic device 100 according to an embodiment may measure the height of the electronic device 100, the movement direction, or an amount of a change in the height through the sensor module included in the electronic device 100.

According to an embodiment, the electronic device 100 may measure the distance between the electronic device 100 and the user through the sensor module included in the electronic device 100. For example, the electronic device 100 may generate a sound wave from a speaker included in the electronic device 100 and measure the distance from the user through a reflected wave reflected and returned from the user's body.

According to various embodiments, the electronic device 100 may measure the distance between the user and the electronic device 100 through a time of flight (TOF) sensor, an optical sensor using various wavelengths, and a camera module.

Referring to FIG. 7B, the electronic device 100 may display the distance between the electronic device 100 and the user included in the first data and the distance between the electronic device 100 and the user included in the second data in the form of a graph 723. The electronic device 100 may display, for example, a reference distance (for example, the distance between the electronic device 100 and the user included in the first data) and a current distance (for example, the distance between the electronic device 100 and the user included in the second data) through the graph 723.

According to an embodiment, the electronic device 100 may reflect a value of the second data varying according to movement of the electronic device 100 to display the graph 723. For example, when a graph 725 indicating the current distance is close to or matches a graph object 727 indicating the reference distance, the electronic device 100 may generate a notification. The electronic device 100 may generate, for example, vibration, a sound, or a change (for example, a change in a color or brightness) in the screen of the distance guide user interface 720 and output a notification.

Referring to FIG. 7C, the electronic device 100 identifies whether an inclination of the electronic device 100 included in the second data acquired through sensor module and an inclination of the electronic device 100 included in the pre-stored first data are included within an allowable range, and when the inclinations are not included within the allowable range, displays an inclination guide user interface 730 for making the second position of the user become the same as the first position of the user on the display 10.

The inclination guide user interface 730 may display, for example, information 731 making a request for tiling the electronic device 100 in a particular direction to the user.

The electronic device 100 according to an embodiment may measure information on an inclination state of the electronic device 100 through the sensor module included in the electronic device 100. For example, the electronic device 100 may measure the inclination of the electronic device 100 through an acceleration sensor, a gyro sensor, and an inclination sensor.

Referring to FIG. 7C, the electronic device 100 may display the inclination of the electronic device 100 included in the first data and the inclination of the electronic device 100 included in the second data in the form of an image 733. The electronic device 100 may display, for example, a reference inclination 737 (for example, the inclination of the electronic device 100 included in the first data) and a current reference 735 (for example, the inclination of the electronic device 100 included in the second data).

According to an embodiment, the electronic device 100 may reflect a value of the second data varying according to movement of the electronic device 100 to display an image. For example, when an inclination state of the image 735 indicating the current inclination is close to or matches an inclination state of the image 737 indicating the reference inclination, the electronic device 100 may generate a notification. The electronic device 100 may generate, for example, vibration, a sound, or a change (for example, a change in a color or brightness) in the screen of the inclination user interface 730 and output a notification.

According to various embodiments, the electronic device 100 may sequentially perform the processes of FIGS. 7A to 7C. However, the disclosure is not limited thereto. For example, after the process of FIG. 7A, the process of FIG. 7C may be performed and the process of FIG. 7B may be performed.

According to various embodiments, when the measurement height is determined and then the height is changed while the distance and the inclination are changed, the electronic device 100 may return to the process of FIG. 7A to adjust the height again. In order to prevent such a situation, the electronic device 100 may generate a vibration or sound notification and guide the user to prevention of a change in the height of the electronic device 100 held by the user from being changed when the height of electronic device 100 leaves the allowable range.

According to various embodiments, the electronic device 100 may display information 740 related to the previously measured BP on the display 10.

According to various embodiments, the electronic device 100 may simultaneously or sequentially display the user interface 710, 720, or 730 and output the sound or vibration corresponding to guide information. The electronic device 100 may output only a sound or vibration corresponding to guide information. For example, the electronic device 100 may output pieces of information 711, 721, and 731 displayed in the user interfaces 710, 720, and 730 through a voice or in various forms of vibration.

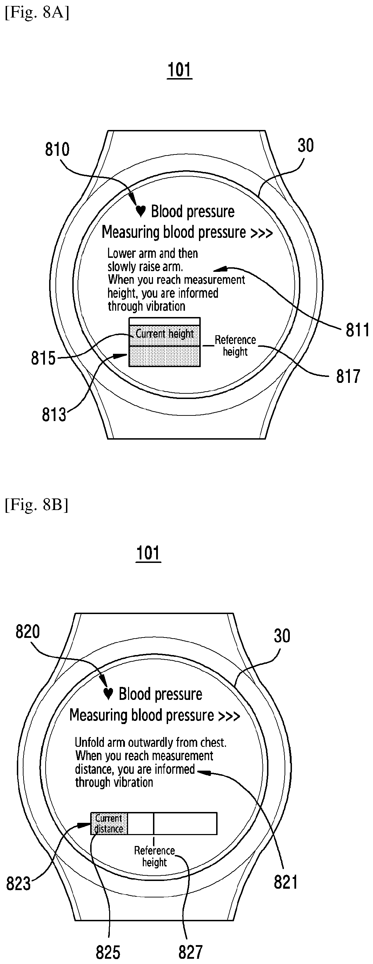

FIGS. 8A and 8B illustrate a situation in which the electronic device guides the user to measurement of biometric information of the user according to an embodiment of the disclosure.

Referring to FIGS. 8A and 8B, the electronic device 101 may be a wearable device worn on the user's wrist. The electronic device 101 may identify the second position of the user at which the BP is measured using second data acquired through the sensor module included in the electronic device 101. The electronic device 101 may identify whether a difference between the first data related to the pre-stored first position of the user and the second data is included within a preset allowable range, and when the different is not within the allowable range, display a user interface including guide information for making the second position of the user become the same as the first position of the user.

Referring to FIG. 8A, the electronic device 101 may identify whether the height of the electronic device 101 included in the second data acquired through sensor module and the height of the electronic device 101 included in the pre-stored first data are included within the allowable range, and when the heights are not included within the allowable range, may display a height guide user interface 810 for making the second position of the user become the same as the first position of the user on the display 30.

The height guide user interface 810 may display information 811 making a request for lowering the arm wearing the electronic device 101 toward bottom and then slowly raising the arm toward the head to the user.

The electronic device 101 according to an embodiment may measure the height of the electronic device 101, a movement direction, or an amount of a change in the height through a sensor module included in the electronic device 101.

The electronic device 101 according to an embodiment may identify the second position of the user on the basis of the second data acquired through the sensor module. The electronic device 101 may display information related to the acquired second data and the pre-stored first data on the display 30.

Referring to FIG. 8A, the electronic device 101 may display the height of the electronic device 101 included in the first data and the height of the electronic device 101 included in the second data in the form of a graph 813. The electronic device 101 may display, for example, a reference height (for example, the height of the electronic device 101 included in the first data) and a current height (for example, the height of the electronic device 101 included in the second data) through the graph 813.

According to an embodiment, the electronic device 101 may reflect a value of the second data varying according to movement of the electronic device 101 to display the graph 813. For example, when a graph object 815 indicating the current height is close to or matches a graph object 817 indicating the reference height, the electronic device 101 may generate a notification. The electronic device 101 may make vibration, a sound, or a change (for example, a change in a color or brightness) in the screen of the height guide user interface 810 and output a notification.

Referring to FIG. 8B, the electronic device 101 may identify whether the distance between the electronic device 101 and the user included in the second data acquired through the sensor module and the distance between the electronic device 101 and the user included in the pre-stored first data are included within a preset allowable range, and when the distances are not included within the allowable range, may display a distance guide user interface 820 for making the second position of the user become the same as the first position of the user on the display 30.

The distance guide user interface 820 may display, for example, information 821 making a request for moving the hand holding the electronic device 101 farther away from the chest to the user.

The electronic device 101 according to an embodiment may measure the height of the electronic device 101, the movement direction, or an amount of a change in the height through the sensor module included in the electronic device 101.

According to an embodiment, the electronic device 101 may measure the distance between the electronic device 101 and the user through the sensor module included in the electronic device 101. For example, the electronic device 101 may generate a sound wave from a speaker included in the electronic device 101 and measure the distance from the user through a reflected wave reflected and returned from the user's body.

Referring to FIG. 8B, the electronic device 101 may display the distance between the electronic device 101 and the user included in the first data and the distance between the electronic device 101 and the user included in the second data in the form of a graph 823. The electronic device 101 may display, for example, a reference distance (for example, the distance between the electronic device 101 and the user included in the first data) and a current distance between (for example, the distance between the electronic device 101 and the user included in the second data) through the graph 823.

According to an embodiment, the electronic device 101 may reflect a value of the second data varying according to movement of the electronic device 101 to display the graph 823. For example, when a graph 825 indicating the current distance is close to or matches a graph object 827 indicating the reference distance, the electronic device 101 may generate a notification. The electronic device 101 may generate, for example, vibration, a sound, or a change (for example, a change in a color or brightness) in the screen of the distance guide user interface 820 and output a notification.

According to various embodiments, the electronic device 101 may simultaneously or sequentially display the user interface 810 or 820 and output the sound or vibration corresponding to guide information. The electronic device 101 may output only a sound or vibration corresponding to guide information. For example, the electronic device 101 may output pieces of information 811 and 821 displayed in the user interfaces 810 and 820 in various forms of vibration.

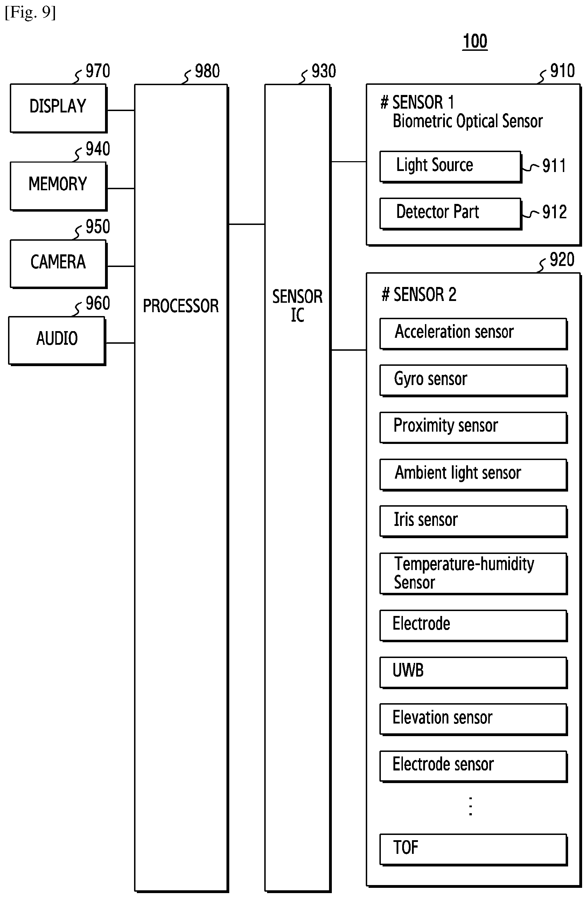

FIG. 9 is a block diagram schematically illustrating the electronic device according to an embodiment of the disclosure.

Referring to FIG. 9, the electronic device 100 may include a first sensor module (sensor 1) 910, a second sensor module (sensor 2) 920, a sensor integrated circuit (IC) 930, a memory 940, a camera module (camera) 950, an audio module (audio) 960, a display 970, and a processor 980. However, the disclosure is not limited thereto. Meanwhile, the block diagram of the electronic device 101 may be similar to the block diagram of the electronic device 100.

According to an embodiment, the first sensor module 910 and the second sensor module 920 may be collectively called a sensor module. The sensor module may include various sensors.

Referring to FIG. 9, the electronic device 100 is divided into the first sensor module 910 and the second sensor module 920, but is not limited thereto.

According to an embodiment, the first sensor module 910 may correspond to a biometric information-measuring optical sensor (for example, the biometric information-measuring optical sensor 20 of FIG. 1).

A light source 911 may include, for example, a light-emitting diode (LED) having a plurality of wavelengths. The electronic device 100 may include one or more emitters according to the plurality of wavelengths. The plurality of wavelengths may include, for example, a green wavelength, a red wavelength, a blue wavelength, and an IR wavelength. However, the wavelengths are not limited thereto.

According to various embodiments, the green wavelength may be used to measure a heart rate. The green wavelength may have an advantage of robust to a noise signal since it permeates through the human's skin shallowly.

According to various embodiments, the red wavelength may deeply permeate through the skin. Accordingly, the electronic device 100 may measure the accurate heart rate through the red wavelength. The electronic device 100 may acquire various pieces of biometric information such as not only the heart rate but also $SPO_2$ using both the red wavelength and the IR wavelength.

According to various embodiments, the electronic device 100 may measure a skin tone through a combination of the red wavelength, the green wavelength, and the IR wavelength.

As described above, the electronic device 100 may measure much more biometric information by adding LEDs having various wavelengths.

According to an embodiment, a detector (detector part) 912 may include one or more photodiodes. The detector 912 may be separated by a preset distance from the light source 911. However, the disclosure is not limited thereto, and the number of detectors 912 may be plural and the plurality of light sources may be separated by different distances from the light source 911.

According to an embodiment, the second sensor module 920 may include sensors for measuring an ECG, a galvanic skin response (GSR), an electroencephalography (EEG), and bioelectric impedance analysis) as well as the optical sensor for measuring biometric information (for example, the biometric information-measuring optical sensor 20 of FIG. 1).

According to various embodiments, the second sensor module 920 may include an acceleration sensor, a proximity sensor, and a gyro sensor for acquiring information related to the location, position, and movement of the electronic device 100. Further, the second sensor module 920 may include a temperature (body temperature) sensor, an iris sensor, and an electrode sensor for acquiring information related to the user.

According to various embodiments, the second sensor module 920 may include a temperature/humidity sensor, an illumination sensor, an IR laser distance-measuring ToF sensor, and an ultra-wideband (UWB) sensor for acquiring information on an external environment of the user.

According to various embodiments, the second sensor module 920 may include a gas detection sensor and a fine dust detection sensor. Further, the second sensor module 920 may further include a communication module for detecting the situation or location of the electronic device 100 such as a GPS or Wi-Fi.

According to an embodiment, the sensor IC 930 may include a sensor deriver controller for directly controlling a sensor and an analog digital converter (ADC).

The sensor driver controller may include, for example, an emitter controller and a detector controller. Accordingly, the sensor driver controller may directly drive an emitter and a detector.

According to various embodiments, the sensor driver controller may serve as an analog front end (AFE). The AFE may include, for example, an ADC for converting a value detected by LED drivers and the detector and a controller for controlling the LED driver and the ADC.

According to various embodiments, a photo input through the detector may be transferred to the processor 980 through a plurality of filters and the ADC, and the processor 980 may extract biometric information which the processor 980 desires to measure and provide the same to the user or store the same in a relevant application.

According to an embodiment, the memory 940 may store information on the user of the electronic device 100. For example, the memory 940 may store at least one piece of information on user's health such as the user's gender, age, height, and weight.

According to various embodiments, the memory 940 may store first data related to the first position. The first data may include, for example, at least one of the height of the electronic device 100, the distance between the electronic device 100 and the user (for example, the user's chest), and the inclination of the electronic device 100. The memory 940 may store BP measured at the first position by the cuff BP monitor.

According to an embodiment, the electronic device 100 may measure ballistocardiogram through the camera module 950. The electronic device 100 may identify the position of the user through the camera module 950. According to various embodiments, the electronic device 100 may measure ballistocardiogram through the acceleration sensor.

According to an embodiment, the electronic device 100 may measure the distance between the electronic device and the electronic device 100 through the audio module 960.

According to an embodiment, the display 970 may provide various guides to the user. For example, the electronic device 100 may display various user interfaces inducing the user to change the position on the display 970.

According to various embodiments, the display 970 may provide the previously measured BP. In this case, the display 970 may display the BP in the form of a numerical value and the form of a graph or a table.

According to an embodiment, the processor 980 may measure the position of the user on the basis of a value input through the sensor and induce the user to take the position which is the same as or similar to the pre-stored reference position for BP measurement.

According to an embodiment, the position of the user may include a position of the user at which the BP can be measured through various sensors included in the electronic device 100. For example, the position of the user may be at least one of the height of the electronic device 100 held by the user, the distance between the electronic device 100 held by the user and the electronic device 100, and the inclination of the electronic device 100 held by the user.

According to various embodiments, position of the user measured by the processor 980 may include a turtle neck position, a crooked back position, distinction between left and right hands, and distinction between standing and sitting positions.

According to an embodiment, the processor 980 may compare the position of the user stored in the memory 940 and the position of the user at which the BP is currently measured. For example, the pre-stored first position of the user (the reference position of BP measurement) and the second position of the user (the position of the user measured as a value currently input through the sensor) may be compared.

According to an embodiment, the processor 980 may compare the height of the electronic device 100 and sequentially compare the distance between the electronic device 100 and the user and the inclination of the electronic device 100.

According to an embodiment, the processor 980 may compare first data and second data, identify whether a comparison result is included in a preset allowable range, and when the comparison result is not included in the allowable range, determine how much the electronic device 100 should be moved or changed to make the second position become the same as the first position.

The first data and the second data included in the preset allowable range mean that, for example, the first data related to the first position and the second data related to the second position are within a relative margin of error from $\pm 1\%$ to 5%. However, the relative margin of error is only an embodiment but is not limited thereto.

According to an embodiment, when the first data and the second data are within the preset allowable range, the processor 980 may measure the BP.

The measurement of the BP may be performed using the PWV type or the PWA type as illustrated in FIGS. 1 to 3. The processor 980 may display information making a request for fixing the position on the display 970 or providing a sound while the BP measurement is performed. When the BP measurement is completed, the electronic device 100 may display health-related information (for example, weight loss) generated in connection with a measurement value, a change, or personal information of the user on the display 970.

According to various embodiments of the disclosure, an electronic device may comprise: a sensor module; a display; a processor operatively connected to the sensor module and the display; and a memory operatively connected to the processor, wherein the memory stores instructions configured to cause the processor to, when executed, obtain first data related to a first position of a user measuring BP through the electronic device, identify a second position of the user measuring BP through the electronic device, based on second data acquired through the sensor module, identify whether a difference between the first data and the second data including at least one of a height of the electronic device and a distance between the electronic device and the user of the electronic device is included in a preset allowable range, and when the difference is not included in the allowable range, display a user interface including guide information for making the second position become equal to the first position through the display.

According to various embodiments, the electronic device may further comprise an audio module and a haptic module, and the memory may store instructions configured to cause the processor to, when executed, simultaneously or sequentially output a sound corresponding to the guide information through the audio module or output vibration corresponding to the guide information through the haptic module when the user interface including the guide information is displayed through the display.

According to various embodiments, the memory may store instructions configured to cause the processor to, when executed, display at least one of a height of the electronic device stored as the first data and a height of the electronic device stored as the second data or a distance between the electronic device and the user stored as the first data and a distance between the electronic device and the user stored as the second data through the display.

According to various embodiments, the electronic device may further comprise an audio module and a haptic module, and the memory may store instructions configured to cause the processor to, when executed, perform at least one of a change in a screen of the user interface, generation of a sound through the audio module, or output of vibration through the haptic module when the difference between the first data and the second data is included in the preset allowable range.

According to various embodiments, each piece of the first data and the second data may further include an inclination of the electronic device.

According to various embodiments, the memory may store instructions configured to cause the processor to, when executed, simultaneously display an inclination of the electronic device stored as the first data and an inclination of the electronic device stored as the second data in the user interface through the display.

According to various embodiments, the electronic device may further comprise an audio module and a haptic module, and the memory may store instructions configured to cause the processor to, when executed, perform at least one of a change in a screen of the user interface, generation of a sound through the audio module, or output of vibration through the haptic module when the difference between the first data and the second data is included in the preset allowable range.

According to various embodiments, the memory may store instructions configured to cause the processor to, when executed, display a user interface including information for acquiring the first data related to the first position through the display.

According to various embodiments, the electronic device may further comprise an audio module and a haptic module, and the memory may store instructions configured to cause the processor to, when executed, simultaneously or sequentially output a sound corresponding to information for generating the first data through the audio module or output vibration corresponding to information for generating the first data when the user interface including the information for acquiring the first data is displayed.

According to various embodiments, wherein the user interface including the information for acquiring the first data may comprise at least one of information for measuring a height of the electronic device, information for measuring a distance between the electronic device and the user, and information for measuring an inclination of the electronic device.

Hereinafter, for convenience of description, the electronic device 100 will be described by way of an example. For example, it is apparent that the following operation description can be applied to the electronic device 101.

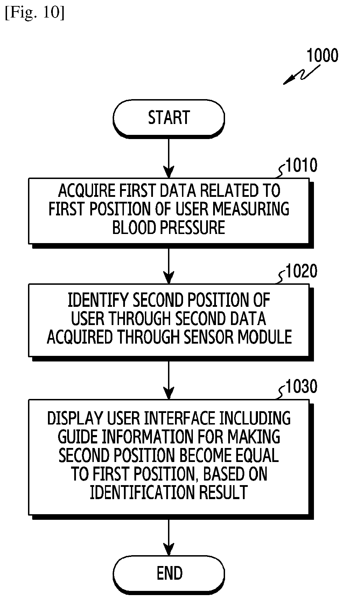

FIG. 10 is a flowchart illustrating a situation in which the electronic device acquires data for measuring biometric information of the user and identifies a position of the user to provide a guide according to an embodiment of the disclosure.

Referring to FIG. 10, in operation 1010 of flowchart 1000 the electronic device 100 may acquire first data related to a first position of the user measuring BP.

According to an embodiment, the electronic device 100 may display a user interface for acquiring first data related to the first position of the user. The electronic device 100 may acquire the first data according to an action by the user of moving or stopping the electronic device 100 in response to the user interface.

In operation 1020, the electronic device 100 may identify the second position of the user on the basis of the second data acquired through sensor module.

According to an embodiment, the second position may be a position identified using the second data acquired through the sensor module included in the electronic device 100 in the state in which BP is measured at the first position and then the user measures the BP again.

In operation 1030, the electronic device 100 may display a user interface including guide information for making the second position become the same as the first position on the basis of the identification result.

According to an embodiment, the electronic device 100 may identify whether a difference between the first data related to the first position and the second data related to the second position is included in a preset allowable range. When the first data and the second data are not included in the allowable range, the electronic device 100 may display a user interface including guide information for making the second position become the same as the first position.

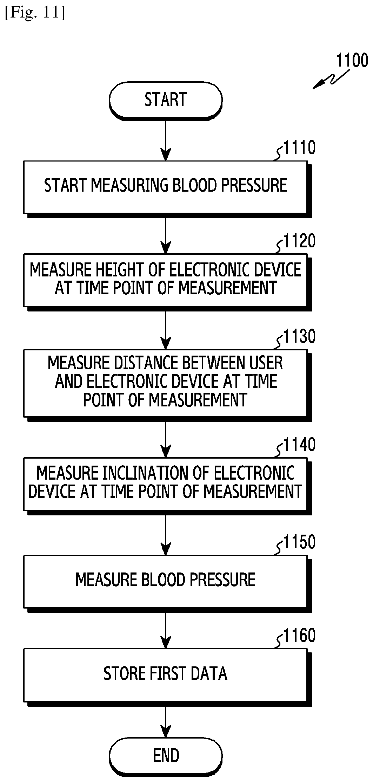

FIG. 11 is a flowchart illustrating a situation in which the electronic device acquires a first position of the user according to an embodiment of the disclosure.

Referring to FIG. 11, at operation 1110 of flowchart 1100 the electronic device 100 may start measuring BP. For example, when a user input making a request for executing a BP measurement application is received, the electronic device 100 may execute the BP application and start measuring the BP.

When the first position of the user is not stored, the electronic device 100 may perform an operation for storing the first position of the user. However, the disclosure is not limited thereto, and the electronic device 100 may perform the operation for storing the first position of the user even when the user input making a request for storing the first position of the user exists.

At operation 1120, the electronic device 100 may measure the height of the electronic device 100 at a time point of the measurement.

According to an embodiment, the electronic device 100 may generate a guide making a request for lowering the arm holding the electronic device 100 as much as possible, raising the arm as much as possible, and then fixing the arm in a comfortable position adjacent to the height of the user's heart to the user. The order in which the arm moves may be reverse.

The electronic device 100 may measure altitude (or air pressure) when the arm is lowered, altitude (or air pressure) when the arm is raised as much as possible, or altitude (or air pressure) of the electronic device 100 at a position at which the user fixes the arm. The electronic device 100 may store the measured value as the first data related to the first position.

At operation 1130, the electronic device 100 may measure the distance between the user and the electronic device 100 at the time point of the measurement.

At operation 1140, the electronic device 100 may measure the inclination of the electronic device 100 at the time point of the measurement.

According to an embodiment, after measuring the height of the electronic device 100 at the first position of the user, the electronic device 100 may measure the reference distance from the user and the inclination. The electronic device 100 may generate a guide making a request for spreading the arm holding the electronic device from the user's chest and then stopping the arm in a comfortable position to the user. Further, the electronic device 100 may generate a guide making a request for changing the inclination while the user holds the electronic device 100 and then stopping in a comfortable position to the user. The electronic device 100 may store the reference distance between the electronic device 100 and the user and the inclination of the electronic device 100 in the state in which the electronic device 100 is fixed as the first data related to the first position through various elements such as the sensor.

At operation 1150, the electronic device 100 may measure BP.

According to an embodiment, the electronic device 100 may measure BP through the PWV type or the PWA type as described in FIG. 3. According to various embodiments, when it is difficult to measure BP since noise is added to the BP measured through the sensor module compared to a preset value, the electronic device 100 may provide a guide to a normal position for BP measurement. In this case, the electronic device 100 may perform again the processes of operations 1120 to 1140.

When BP is measured, the electronic device 100 may store the first data related to the first position (for example, the height of the electronic device 100, the distance between the electronic device 100 and the user, and the inclination of the electronic device 100) in operation 1160. According to various embodiments, the electronic device 100 may perform the processes of operations 1120 to 1150 a plurality of times.

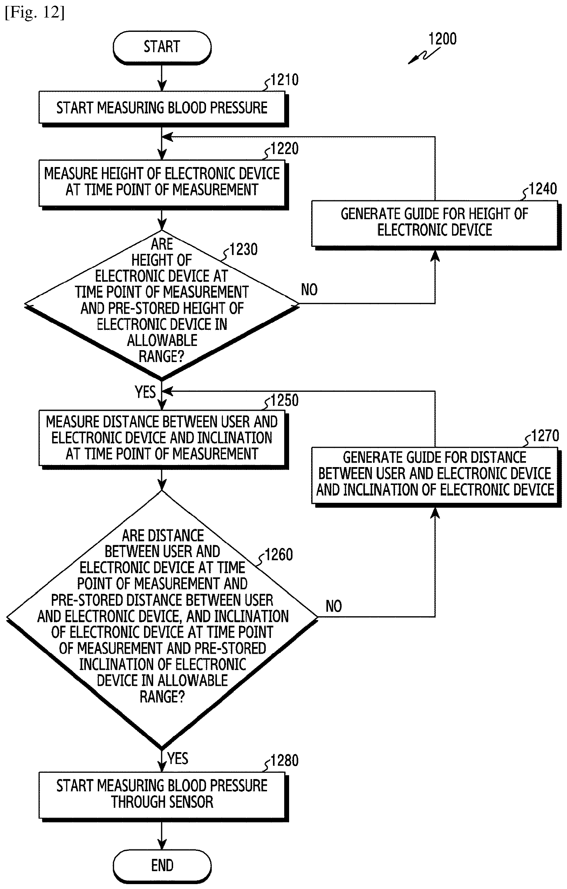

FIG. 12 is a flowchart illustrating a situation in which the electronic device guides the user to take a measurement position when BP is measured through the electronic device according to an embodiment of the disclosure.

Referring to FIG. 12, at operation 1210 of flowchart 1200 the electronic device 100 may measure BP. For example, when a user input making a request for executing a BP measurement application is received, the electronic device 100 may execute the BP application and start measuring the BP.

At operation 1220, the electronic device 100 may measure the height of the electronic device 100 at a time point of the measurement.

According to an embodiment, the electronic device 100 may store data input through the sensor in the state in which movement of the electronic device 100 stops as second data.

At operation 1230, the electronic device 100 may identify whether the height of the electronic device at the time point of the measurement and the pre-stored height of the electronic device are included in a preset allowable range at operation 1230. For example, the electronic device 100 may compare the pre-stored first data with input second data and identify whether a difference between the current height and the stored height is within a preset range.

When the difference leaves the preset allowable range, the electronic device 100 may generate a guide to the height of the electronic device 100 to make a request for changing the height of the electronic device 100 to the user in operation 1240.

When the difference is within the preset allowable range, the electronic device 100 may measure the distance between the user and the electronic device 100 at the time point of the measurement and the inclination of the electronic device 100 in operation 1250. In this case, the electronic device 100 may generate a guide for limiting movement of the user in order to measure the distance between the electronic device 100 and the user and the inclination of the electronic device 100 at the current height without any movement of the electronic device 100. For example, when the height of the electronic device 100 is changed to leave the allowable range, the electronic device 100 may generate vibration or an alarm through a sound.

According to an embodiment, the electronic device 100 may store data input through the sensor in the state in which movement of the electronic device 100 stops as second data.

At operation 1260, the electronic device 100 may identify whether the distance between the electronic device 100 and the user at the time point of the measurement and the pre-stored distance between the electronic device 100 and the user, and the inclination of the electronic device 100 and the pre-stored inclination of the electronic device 100 are included within preset allowable ranges.

For example, the electronic device 100 may compare the pre-stored first data within the input second data and identify whether the distance from the current user of the electronic device 100 is within a preset range from the stored distance and whether the current inclination of the electronic device 100 is within a preset range from the stored inclination.

At operation 1270, when the distance and the inclination leave the allow ranges, the electronic device 100 may generate a guide making a request for changing the distance between the electronic device 100 and the user and the inclination of the electronic device 100 to the user.

When the distance and the inclination are within the allowable ranges, the electronic device 100 may measure BP through the sensor in operation 1280. According to various embodiments, the electronic device 100 may derive the accurate BP by correcting the measured BP through the correction value described in FIG. 5.

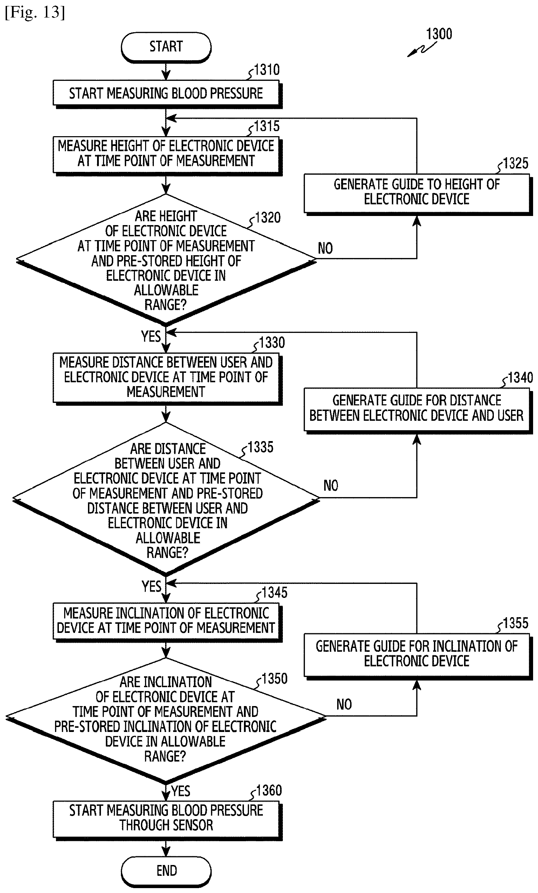

FIG. 13 is a flowchart illustrating another situation in which the electronic device guides the user to take a measurement position when BP is measured through the electronic device according to various embodiments of the disclosure.

Referring to FIG. 13, an embodiment 1300 in which the electronic device 100 measures the height of the electronic device 100, measures the distance between the user and the electronic device 100, and then measures the inclination of the electronic device 100. A part different from that of FIG. 12 will be described. Operations 1310, 1315, 1320, and 1325 may be the same as operations 1210, 1220, 1230, and 1240 of FIG. 12.

At operation 1330, the electronic device 100 may measure the distance between the user and the electronic device 100 at the time point of the measurement.

At operation 1335, the electronic device 100 may identify whether the distance between the electronic device 100 and the user at the time point of the measurement and the pre-stored distance between the electronic device 100 and the user are included in a preset allowable range in operation 1335.

When the distance leaves the allowable range, the electronic device 100 may generate a guide to the distance between the electronic device 100 and the user to make a request for changing the distance between the electronic device 100 and the user to the user in operation 1340.

When the distance is within the allowable range, the electronic device 100 may measure the inclination of the electronic device 100 at the time point of the measurement at operation 1345.

At operation 1350, the electronic device 100 may identify whether the inclination of the electronic device 100 at the time point of the measurement and the pre-stored inclination of the electronic device 100 are included within the preset allowable range.

When the inclination leaves the allowable range, the electronic device 100 may generate a guide to the inclination of the electronic device 100 to make a request for changing the inclination of the electronic device 100 to the user in operation 1355.

When the distance and the inclination are within the allowable ranges, the electronic device 100 may measure BP through the sensor in operation 1360.

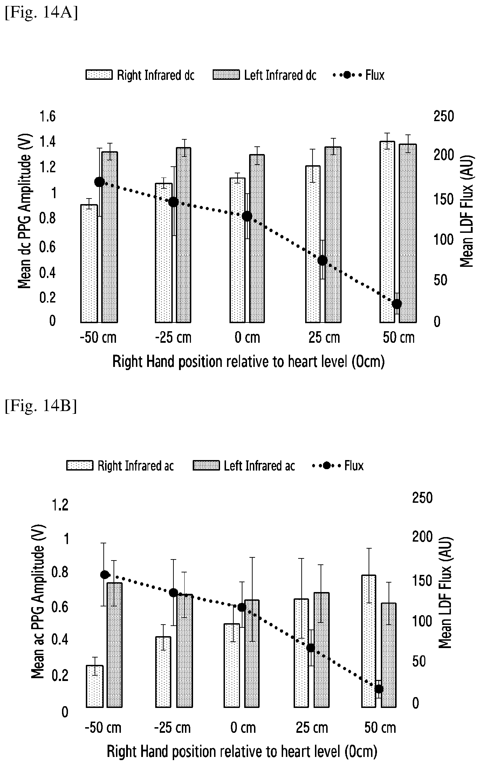

FIGS. 14A and 14B illustrate a situation in which the margin of error is generated when a reference position of BP measurement pre-stored in the electronic device does not match a user's position of BP measurement according to an embodiment of the disclosure.

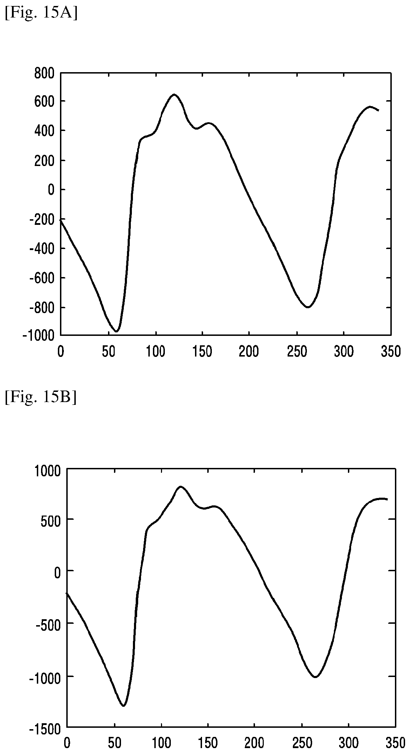

FIGS. 15A and 15B illustrate a situation in which the margin of error is generated when a reference position of BP measurement pre-stored in the electronic device does not match a user's position of BP measurement according to an embodiment of the disclosure.

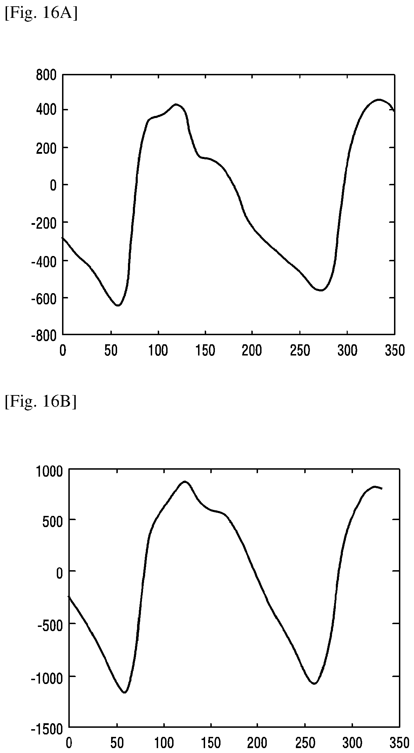

FIGS. 16A and 16B illustrate a situation in which the margin of error is generated when a reference position of BP measurement pre-stored in the electronic device does not match a user's position of BP measurement according to an embodiment of the disclosure.

FIGS. 14A and 14B are graphs illustrating a change in direct current (DC) and alternating current (AC) according to a position of the user for BP measurement. The change in DC and AC may be an index indicating an influence of photoplethysmogram, pulse wave (PPG) varying according to the position of the user for BP measurement.

Referring to FIGS. 14A and 14B, an x axis of the graph may be the height of the electronic device 100. The right part from 0 cm on the x axis may mean that the electronic device 100 is above the heart and the left part may mean that the electronic device 100 is below the heart.

Bar graphs on a Y axis may indicate DC and AC, respectively, and a dotted line may be a flux of blood.

Referring to FIGS. 14A and 14B, when the hand holding the electronic device 100 becomes above the heart, force for making the flux of blood flows to fingertips may be smaller due to an influence of gravity.

Referring to FIG. 15A, FIG. 15A may show a change (Y axis) in an amount of detection by the biometric information-measuring optical sensor according to a change (X axis) in time at various positions of the user, 15B may show a change (Y axis) in an amount of detection by the biometric information-measuring optical sensor according to a change (X axis) in time at various positions of the user, 16A may show a change (Y axis) in an amount of detection by the biometric information-measuring optical sensor according to a change (X axis) in time at various positions of the user, and 16B may show a change (Y axis) in an amount of detection by the biometric information-measuring optical sensor according to a change (X axis) in time at various positions of the user.

FIG. 15A shows a measurement result in the state which the user puts the hand on the desk and unfolds the wrist while the user is sitting.

Referring to FIG. 15B, FIG. 15B shows a measurement result in the state in which the user puts the hand on the desk and folds the wrist while the user is sitting. Although shapes of the graphs are similar but the variation of the amount of detection by the biometric information-measuring optical sensor is different, so that different BPs may be finally derived.

Referring to FIG. 16A, FIG. 16A shows a measurement result in the state in which the user lowers the hand and unfolds the wrist while the user is sitting.

Referring to FIG 16B, FIG. 16B shows a measurement result in the state in which the user lowers the hand and folds the wrist while the user is sitting. Since shapes of the graphs are different and also the variation of the amount of detection by the biometric information-measuring optical sensor is different, different BPs may be finally derived.

As described above, the electronic device 100 according to various embodiments of the disclosure may store the reference position for BP measurement and, when the user desires to measure BP, provide various guide methods of guiding the user to the reference position in order to reduce the margin of error according to a position for BP measurement.

According to various embodiments of the disclosure, when BP is measured, the electronic device may guide to measurement of BP at a height which is the same as or similar to a height pre-stored in the electronic device. According to various embodiments of the disclosure, when BP is measured, the electronic device may guide to measurement of BP in a distance which is the same as or similar to a distance to the user pre-stored in the electronic device. According to various embodiments of the disclosure, when BP is measured, the electronic device may guide to measurement of BP at an inclination which is the same as or similar to an inclination pre-stored in the electronic device.

According to various embodiments of the disclosure, a method of controlling an electronic device may comprise obtaining first data related to a first position of a user measuring BP through the electronic device; identifying a second position of the user measuring BP through the electronic device, based on second data acquired through a sensor module; identifying whether a difference between the first data and the second data including at least one of a height of the electronic device and a distance between the electronic device and the user of the electronic device is included in a preset allowable range; and displaying a user interface including guide information for making the second position become equal to the first position, based on a result of the identification.

According to various embodiments, the method may further comprise simultaneously or sequentially outputting a sound or vibration corresponding to the guide information when the user interface including the guide information is displayed.

According to various embodiments, the method may further comprise displaying at least one of a height of the electronic device stored as the first data and a height of the electronic device stored as the second data or a distance between the electronic device and the user stored as the first data and a distance between the electronic device and the user stored as the second data.

According to various embodiments, the method may further comprise performing at least one of a change in a screen of the user interface, generation of a sound through the audio module, or output of vibration, based on a result of the identification.

According to various embodiments, each piece of the first data and the second data may further include an inclination of the electronic device.

According to various embodiments, the method may further comprise displaying an inclination of the electronic device stored as the first data and an inclination of the electronic device stored as the second data.

According to various embodiments, the method may further comprise performing at least one of a change in a screen of the user interface, generation of a sound, or output of vibration, based on a result of the identification.

According to various embodiments, the method may further comprise displaying a user interface including guide information for acquiring the first data related to the first position.

According to various embodiments, the method may further comprise simultaneously or sequentially outputting a sound or vibration corresponding to information for generating the first data related to the first position when the user interface including the information for acquiring the first data related to the first position is displayed.

According to various embodiments of the disclosure, a computer program product comprising a computer-readable recording medium storing instructions for executing operations in a computer may comprise: obtaining first data related to a first position of a user measuring BP through the electronic device; identifying a second position of the user measuring BP through the electronic device, based on second data acquired through a sensor module; identifying whether a difference between the first data and the second data including at least one of a height of the electronic device and a distance between the electronic device and the user of eh electronic device is included in a preset allowable range; and displaying a user interface including guide information for making the second position become equal to the first position, based on a result of the identification.

The electronic device according to various embodiments may be one of various types of electronic devices. The electronic devices may include, for example, a portable communication device (e.g., a smartphone), a computer device, a portable multimedia device, a portable medical device, a camera, a wearable device, or a home appliance. According to an embodiment of the disclosure, the electronic devices are not limited to those described above.

It should be appreciated that various embodiments of the disclosure and the terms used therein are not intended to limit the technological features set forth herein to particular embodiments and include various changes, equivalents, or replacements for a corresponding embodiment. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include any one of, or all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively," as "coupled with," "coupled to," "connected with," or "connected to" another element (e.g., a second element), it means that the element may be coupled with the other element directly (e.g., wired), wirelessly, or via a third element.

As used herein, the term "module" may include a unit implemented in hardware, software, or firmware, and may interchangeably be used with other terms, for example, "logic," "logic block," "part," or "circuitry." A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 340) including one or more instructions that are stored in a storage medium (e.g., internal memory 336 or external memory 338) that is readable by a machine (e.g., the electronic device 301). For example, a processor (e.g., the processor 320) of the machine (e.g., the electronic device 301) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an embodiment, a method according to various embodiments of the disclosure may be included and provided in a computer program product. The computer program product may be traded as a product between a seller and a buyer. The computer program product may be distributed in the form of a machine-readable storage medium (e.g., compact disc read only memory (CD-ROM)), or be distributed (e.g., downloaded or uploaded) online via an application store (e.g., PlayStore™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

While the disclosure has been shown and described with reference to various embodiments thereof, it will be understood by those skilled in the art that various changes in form and details may be made therein without departing from the scope of the invention as defined by the appended claims.

## Claims

1. An electronic device comprising:

   a sensor module (20);
   a display (10);
   a processor (320) operatively connected to the sensor module (20) and the display (10); and
   a memory (330) storing instructions which, when executed by said processor, configure the processor (320) to:

      obtain first data related to a first position of a user measuring blood pressure (BP) through the electronic device,
identify a second position of the user measuring the BP, based on second data acquired through the sensor module (20),
wherein each of the first data and the second data includes an inclination of the electronic device,
identify whether a difference between the first data and the second data, including a difference in the inclination of the electronic device and a difference in at least one of a height of

the electronic device or a distance between the electronic device and the user, is included in a preset range, and

when the difference is not included in the range, display a user interface, including guide information for adjusting the second position to become equal to the first position, through the display,
wherein the guide information includes a first graph object (717, 727, 737, 817, 827) indicating a reference position and corresponding to the first position, and a second graph object (715, 725, 735, 815, 825) indicating a current position and corresponding to the second position.

2. The electronic device of claim 1, further comprising an audio module and a haptic module, wherein the memory further stores instructions which, when executed, further configure the processor to concurrently or sequentially output a sound corresponding to the guide information through the audio module or output a vibration corresponding to the guide information through the haptic module when the guide information is displayed.

3. The electronic device of claim 1, wherein the memory further stores instructions which, when executed, further configure the processor to display at least one of:

a height of the electronic device stored as the first data and a height of the electronic device stored as the second data, or
a distance between the electronic device and the user stored as the first data and a distance between the electronic device and the user stored as the second data.

4. The electronic device of claim 1,

further comprising:

an audio module; and
a haptic module,

wherein the memory further stores instructions which, when executed, further configure the processor to perform at least one of change a screen of the user interface, generate a sound through the audio module, or output a vibration through the haptic module, when the difference is included in the range.

5. The electronic device of claim 1, wherein the memory further stores instructions which, when executed, further configure the processor to simultaneously display a first inclination of the electronic device stored as the first data and a second inclination of the electronic device stored as the second data in the user interface.

6. The electronic device of claim 1, wherein the memory further stores instructions which, when executed, further configure the processor to:
display the user interface including information for acquiring the first data; and concurrently or sequentially output a sound corresponding to information for generating the first data through an audio module or output vibration corresponding to information for generating the first data through a haptic module when the information for acquiring the first data is displayed.

7. The electronic device of claim 6, wherein the information for acquiring the first data comprises at least one of information for measuring a height of the electronic device, information for measuring a distance between the electronic device and the user, or information for measuring an inclination of the electronic device.

8. A processor-implemented method of controlling an electronic device, the method comprising:

obtaining first data related to a first position of a user measuring blood pressure (BP) through the electronic device (1010);

identifying a second position of the user measuring the BP, based on second data acquired through a sensor module (1020);
wherein each of the first data and the second data further includes an inclination of the electronic device;
identifying whether a difference between the first data and the second data, including the inclination of the electronic device and at least one of a height of the electronic device or a distance between the electronic device and the user, is included in a preset range; and
displaying a user interface, including guide information for adjusting the second position to become equal to the first position, based on a result of the identification (1030), and further comprising displaying a first inclination of the electronic device stored as the first data and a second inclination of the electronic device stored as the second data,
wherein the guide information includes a first graph object indicating a reference position and corresponding to the first position, and a second graph object indicating a current position and corresponding to the second position.

9. The method of claim 8, further comprising concurrently or sequentially outputting a sound or vibration corresponding to the guide information when the guide information is displayed.

10. The method of claim 8, further comprising displaying at least one of a height of the electronic device stored as the first data and a height of the electronic device stored as the second data, or a distance between the electronic device and the user stored as the first data and a distance between the electronic device and the user stored as the second data.

11. The method of claim 8, further comprising at least one of changing a screen of the user interface, generating a sound through an audio module, or outputting a vibration, based on a result of the identification.

12. The method of claim 8, further comprising:

displaying the user interface including guide information for acquiring the first data; and
concurrently or sequentially outputting a sound or vibration corresponding to information for generating the first data when the information for acquiring the first data is displayed.

13. A computer program product comprising a non-transitory computer-readable recording medium storing instructions which, when the program is executed by the processor of a device, cause the processor to perform the operations comprising:

obtaining first data related to a first position of a user measuring blood pressure (BP) through the device (1010);
identifying a second position of the user measuring the BP, based on second data acquired through a sensor module (1020);
wherein each of the first data and the second data further includes an inclination of the device;
identifying whether a difference between the first data and the second data, including the inclination of the device and at least one of a height of the electronic device or a distance between the device and the user, is included in a preset range; and
displaying a user interface, including guide information for adjusting the second position to become equal to the first position, based on a result of the identification (1030), and further comprising displaying a first inclination of the electronic device stored as the first data and a second inclination of the electronic device stored as the second data,
wherein the guide information includes a first graph object indicating a reference position and corresponding to the first position, and a second graph object indicating a current position and corresponding to the second position.

**Patentansprüche**

1. Elektronische Vorrichtung, die Folgendes umfasst:

ein Sensormodul (20);
eine Anzeige (10);
einen Prozessor (320), der betriebsmäßig mit dem Sensormodul (20) und der Anzeige (10) verbunden ist; und

einen Speicher (330), in dem Anweisungen gespeichert sind, die, wenn sie von dem Prozessor ausgeführt werden, den Prozessor (412) zu Folgendem konfigurieren:

Erhalten von ersten Daten, die sich auf eine erste Position eines Benutzers beziehen, der seinen Blutdruck (BP) misst, über die elektronische Vorrichtung,

Identifizieren einer zweiten Position des Benutzers, der seinen Blutdruck misst, basierend auf zweiten Daten, die über das Sensormodul (20) erfasst werden,

wobei sowohl die ersten Daten als auch die zweiten Daten eine Neigung der elektronischen Vorrichtung enthalten,

Identifizieren, ob eine Differenz zwischen den ersten Daten und den zweiten Daten, einschließlich einer Differenz bezüglich der Neigung der elektronischen Vorrichtung und einer Differenz bezüglich mindestens einem von einer Höhe der elektronischen Vorrichtung oder einem Abstand zwischen der elektronischen Vorrichtung und dem Benutzer in einem voreingestellten Bereich enthalten ist, und

wenn die Differenz nicht in dem Bereich enthalten ist, Anzeigen einer Benutzerschnittstelle, die Führungsinformationen zum Anpassen der zweiten Position enthält, damit sie gleich der ersten Position wird, über die Anzeige,

wobei die Führungsinformationen ein erstes Grafikobjekt (717, 727, 737, 817, 827) enthalten, das auf eine Referenzposition hinweist und der ersten Position entspricht, und ein zweites Grafikobjekt (715, 725, 735, 815, 825), das auf eine aktuelle Position hinweist und der zweiten Position entspricht.

2. Elektronische Vorrichtung nach Anspruch 1, die ferner ein Audiomodul und ein haptisches Modul umfasst, wobei in dem Speicher ferner Anweisungen gespeichert sind, die, wenn sie ausgeführt werden, den Prozessor dazu konfigurieren, gleichzeitig oder nacheinander einen den Führungsinformationen entsprechenden Ton über das Audiomodul auszugeben oder eine den Führungsinformationen entsprechende Vibration über das haptische Modul auszugeben, wenn die Führungsinformationen angezeigt werden.

3. Elektronische Vorrichtung nach Anspruch 1, wobei in dem Speicher ferner Anweisungen gespeichert sind, die, wenn sie ausgeführt werden, den Prozessor so konfigurieren, dass er mindestens eines von Folgendem anzeigt:

eine Höhe der elektronischen Vorrichtung, die als die ersten Daten gespeichert ist, und eine Höhe der elektronischen Vorrichtung, die als die zweiten Daten gespeichert ist, oder

eine Entfernung zwischen der elektronischen Vorrichtung und dem Benutzer, die als die ersten Daten gespeichert ist, und eine Entfernung zwischen der elektronischen Vorrichtung und dem Benutzer, die als die zweiten Daten gespeichert ist.

4. Elektronische Vorrichtung nach Anspruch 1,
ferner umfassend:

ein Audiomodul; und

ein haptisches Modul,

wobei in dem Speicher ferner Anweisungen gespeichert sind, die, wenn sie ausgeführt werden, den Prozessor so konfigurieren, dass er mindestens einen der folgenden Vorgänge ausführt: Ändern eines Bildschirms der Benutzerschnittstelle, Erzeugen eines Tons durch das Audiomodul oder Ausgeben einer Vibration durch das haptische Modul, wenn die Differenz in dem Bereich enthalten ist.

5. Elektronische Vorrichtung nach Anspruch 1, wobei in dem Speicher ferner Anweisungen gespeichert sind, die, wenn sie ausgeführt werden, den Prozessor so konfigurieren, dass er gleichzeitig eine erste Neigung der elektronischen Vorrichtung, die als die ersten Daten gespeichert ist, und eine zweite Neigung der elektronischen Vorrichtung, die als die zweiten Daten gespeichert ist, in der Benutzerschnittstelle anzeigt.

6. Elektronische Vorrichtung nach Anspruch 1, wobei in dem Speicher ferner Anweisungen gespeichert sind, die, wenn sie ausgeführt werden, den Prozessor ferner zu Folgendem konfigurieren: Anzeigen der Benutzerschnittstelle, die Informationen zum Erfassen der ersten Daten enthält; und gleichzeitiges oder nacheinander erfolgendes Ausgeben eines Tons, der Informationen zum Erzeugen der ersten Daten entspricht, durch ein Audiomodul, oder Ausgeben einer Vibration, die Informationen zum Erzeugen der ersten Daten entspricht, durch ein haptisches Modul, wenn die Informationen zum Erfassen der ersten Daten angezeigt werden.

7. Elektronische Vorrichtung nach Anspruch 6, wobei die Informationen zum Erfassen der ersten Daten mindestens eine der folgenden Informationen umfassen: Informationen zum Messen einer Höhe der elektronischen Vorrichtung, Informationen zum Messen eines Abstands zwischen der elektronischen Vorrichtung und dem Benutzer oder Informationen zum Messen einer Neigung der elektronischen Vorrichtung.

8. Prozessor-implementiertes Verfahren zum Steuern einer elektronischen Vorrichtung, wobei das Verfahren Folgendes umfasst:

Erhalten von ersten Daten, die sich auf eine erste Position eines Benutzers beziehen, der seinen Blutdruck (BP) misst, über die elektronische Vorrichtung (1010);
Identifizieren einer zweiten Position des Benutzers, der seinen Blutdruck misst, basierend auf zweiten Daten, die über ein Sensormodul erhalten werden (1020);
wobei sowohl die ersten Daten als auch die zweiten Daten ferner eine Neigung der elektronischen Vorrichtung enthalten;
Identifizieren, ob eine Differenz zwischen den ersten Daten und den zweiten Daten, einschließlich der Neigung der elektronischen Vorrichtung und mindestens einem von einer Höhe der elektronischen Vorrichtung oder einem Abstand zwischen der elektronischen Vorrichtung und dem Benutzer, in einem voreingestellten Bereich enthalten ist, und
Anzeigen einer Benutzerschnittstelle, die Führungsinformationen zum Anpassen der zweiten Position enthält, so dass diese gleich der ersten Position wird, basierend auf einem Ergebnis der Identifizierung (1030), und ferner umfassend das Anzeigen einer ersten Neigung der elektronischen Vorrichtung, die als die ersten Daten gespeichert ist, und einer zweiten Neigung der elektronischen Vorrichtung, die als die zweiten Daten gespeichert ist,
wobei die Führungsinformationen ein erstes Grafikobjekt enthalten, das auf eine Referenzposition hinweist und der ersten Position entspricht, und ein zweites Grafikobjekt, das auf eine aktuelle Position hinweist und der zweiten Position entspricht.

9. Verfahren nach Anspruch 8, das ferner das gleichzeitige oder nacheinander erfolgende Ausgeben eines Tons oder einer Vibration umfasst, der bzw. die den Führungsinformationen entspricht, wenn die Führungsinformationen angezeigt werden.

10. Verfahren nach Anspruch 8, ferner umfassend das Anzeigen mindestens eines der Folgenden: einer Höhe der elektronischen Vorrichtung, die als die ersten Daten gespeichert ist, und einer Höhe der elektronischen Vorrichtung, die als die zweiten Daten gespeichert ist, oder eines Abstands zwischen der elektronischen Vorrichtung und dem Benutzer, der als die ersten Daten gespeichert ist, und eines Abstands zwischen der elektronischen Vorrichtung und dem Benutzer, der als die zweiten Daten gespeichert ist.

11. Verfahren nach Anspruch 8, das ferner mindestens eines der folgenden Vorgänge umfasst: Ändern eines Bildschirms der Benutzerschnittstelle, Erzeugen eines Tons durch ein Audiomodul oder Ausgeben einer Vibration, basierend auf einem Ergebnis der Identifizierung.

12. Verfahren nach Anspruch 8, das ferner Folgendes umfasst:

Anzeigen der Benutzerschnittstelle, die Führungsinformationen zum Erfassen der ersten Daten einschließt; und gleichzeitiges oder nacheinander erfolgendes Ausgeben eines Tons oder einer Vibration entsprechend Informationen zum Erzeugen der ersten Daten, wenn die Informationen zum Erfassen der ersten Daten angezeigt werden.

13. Computerprogrammprodukt, das ein nicht flüchtiges, computerlesbares Aufzeichnungsmedium umfasst, in dem Anweisungen gespeichert sind, die, wenn das Programm durch den Prozessor einer Vorrichtung ausgeführt wird, den Prozessor veranlassen, die folgenden Vorgänge durchzuführen:

Erhalten von ersten Daten, die sich auf eine erste Position eines Benutzers beziehen, der seinen Blutdruck (BP) misst, über die Vorrichtung (1010);
Identifizieren einer zweiten Position des Benutzers, der seinen Blutdruck misst, basierend auf zweiten Daten, die über ein Sensormodul erfasst werden (1020);
wobei sowohl die ersten Daten als auch die zweiten Daten ferner eine Neigung der Vorrichtung enthalten;
Identifizieren, ob eine Differenz zwischen den ersten Daten und den zweiten Daten, einschließlich der Neigung der Vorrichtung und mindestens einem von einer Höhe der elektronischen Vorrichtung oder einem Abstand zwischen der Vorrichtung und dem Benutzer, in einem voreingestellten Bereich enthalten ist, und Anzeigen einer Benutzerschnittstelle, die Führungsinformationen zum Anpassen der zweiten Position enthält, so dass diese gleich der ersten Position wird, basierend auf einem Ergebnis der Identifizierung (1030), und ferner umfassend das Anzeigen einer ersten Neigung der elektronischen Vorrichtung, die als die ersten Daten gespeichert ist, und einer zweiten Neigung der elektronischen Vorrichtung, die als die zweiten Daten gespeichert ist,

wobei die Führungsinformationen ein erstes Grafikobjekt enthalten, das auf eine Referenzposition hinweist und der ersten Position entspricht, und ein zweites Grafikobjekt, das auf eine aktuelle Position hinweist und der zweiten Position entspricht.

**Revendications**

1.  Appareil électronique comprenant :

    un module capteur (20),
    un écran (10),
    un processeur (320) relié de manière fonctionnelle au module capteur (20) et à l'écran (10), et
    une mémoire (330) stockant des instructions qui, lorsqu'elles sont exécutées par ledit processeur, configurent le processeur (320) pour permettre de :

    obtenir des premières données liées à une première position d'un utilisateur mesurant sa pression artérielle (PA) grâce à l'appareil électronique,
    identifier une deuxième position de l'utilisateur mesurant sa PA, compte tenu de deuxièmes données acquises grâce au module capteur (20),
    lesdites premières données et deuxièmes données renfermant respectivement une inclinaison de l'appareil électronique,
    identifier si une différence entre les premières données et les deuxièmes données, y compris une différence concernant l'inclinaison de l'appareil électronique ainsi qu'une différence concernant la hauteur à laquelle se trouve l'appareil électronique et/ou la distance entre l'appareil électronique et l'utilisateur, est comprise dans un intervalle prédéfini, et
    lorsque la différence n'est pas présente dans ledit intervalle, afficher, par le biais de l'écran, une interface utilisateur comprenant des informations de guidage permettant d'ajuster la deuxième position de façon qu'elle soit identique à la première position,
    lesdites informations de guidage comprenant un premier objet graphique (717, 727, 737, 817, 827) indiquant une position de référence et correspondant à la première position, et un deuxième objet graphique (715, 725, 735, 815, 825) indiquant une position actuelle et correspondant à la deuxième position.

2.  Appareil électronique selon la revendication 1, comprenant en outre un module audio et un module haptique, ladite mémoire stockant en outre des instructions qui, lorsqu'elles sont exécutées, configurent en outre le processeur pour permettre d'émettre simultanément ou successivement un son correspondant aux informations de guidage par le biais du module audio ou une vibration correspondant aux informations de guidage par le biais du module haptique lorsque les informations de guidage sont affichées.

3.  Appareil électronique selon la revendication 1, dans lequel la mémoire stocke en outre des instructions qui, lorsqu'elles sont exécutées, configurent en outre le processeur pour permettre d'afficher au moins l'un des éléments suivants :

    la hauteur à laquelle se trouve l'appareil électronique stockée en tant que premières données et la hauteur à laquelle se trouve l'appareil électronique stockée en tant que deuxièmes données, et
    la distance entre l'appareil électronique et l'utilisateur stockée en tant que premières données et la distance entre l'appareil électronique et l'utilisateur stockée en tant que deuxièmes données.

4.  Appareil électronique selon la revendication 1,

    comprenant en outre :

    un module audio, et

un module haptique,

ladite mémoire stockant en outre des instructions qui, lorsqu'elles sont exécutées, configurent en outre le processeur pour permettre d'effectuer au moins l'une des opérations suivantes : modifier un visuel de l'interface utilisateur, générer un son par le biais du module audio et émettre une vibration par le biais du module haptique, lorsque la différence est comprise dans ledit intervalle.

5. Appareil électronique selon la revendication 1, dans lequel la mémoire stocke en outre des instructions qui, lorsqu'elles sont exécutées, configurent en outre le processeur pour permettre d'afficher simultanément, dans l'interface utilisateur, une première inclinaison de l'appareil électronique stockée en tant que premières données et une deuxième inclinaison de l'appareil électronique stockée en tant que deuxièmes données.

6. Appareil électronique selon la revendication 1, dans lequel la mémoire stocke en outre des instructions qui, lorsqu'elles sont exécutées, configurent en outre le processeur pour permettre de :
afficher l'interface utilisateur qui comprend des informations permettant d'acquérir les premières données, et émettre simultanément ou séquentiellement un son correspondant à des informations permettant de générer les premières données par le biais d'un module audio ou une vibration correspondant à des informations permettant de générer les premières données par le biais d'un module haptique lorsque les informations permettant d'acquérir les premières données sont affichées.

7. Appareil électronique selon la revendication 6, dans lequel les informations permettant d'acquérir les premières données comprennent au moins un type d'informations parmi les suivantes : informations permettant de mesure la hauteur à laquelle se trouve l'appareil électronique, informations permettant de mesurer la distance entre l'appareil électronique et l'utilisateur, et informations permettant de mesurer l'inclinaison de l'appareil électronique.

8. Procédé mis en oeuvre par un processeur, permettant de commander un appareil électronique, le procédé comprenant les opérations consistant à :

obtenir des premières données liées à une première position d'un utilisateur mesurant sa pression artérielle (PA) grâce à l'appareil électronique (1010),
identifier une deuxième position de l'utilisateur mesurant sa PA, compte tenu de deuxièmes données acquises par le biais d'un module capteur (1020),
lesdites premières données et deuxièmes données renfermant respectivement en outre une inclinaison de l'appareil électronique,
identifier si une différence entre les premières données et les deuxièmes données, y compris l'inclinaison de l'appareil électronique ainsi que la hauteur à laquelle se trouve l'appareil électronique et/ou la distance entre l'appareil électronique et l'utilisateur, est comprise dans un intervalle prédéfini, et
afficher une interface utilisateur, comprenant des informations de guidage permettant d'ajuster la deuxième position de façon qu'elle soit identique à la première position, compte tenu du résultat de l'identification (1030),
ce qui comprend en outre l'affichage d'une première inclinaison de l'appareil électronique stockée en tant que premières données et d'une deuxième inclinaison de l'appareil électronique stockée en tant que deuxièmes données,
lesdites informations de guidage comprenant un premier objet graphique indiquant une position de référence et correspondant à la première position, et un deuxième objet graphique indiquant une position actuelle et correspondant à la deuxième position.

9. Procédé selon la revendication 8, comprenant en outre l'émission simultanée ou successive d'un son ou d'une vibration correspondant aux informations de guidage lorsque les informations de guidage sont affichées.

10. Procédé selon la revendication 8, comprenant en outre l'affichage d'au moins l'un des éléments suivants : la hauteur à laquelle se trouve l'appareil électronique stockée en tant que premières données et la hauteur à laquelle se trouve l'appareil électronique stockée en tant que deuxièmes données, et la distance entre l'appareil électronique et l'utilisateur stockée en tant que premières données et la distance entre l'appareil électronique et l'utilisateur stockée en tant que deuxièmes données.

11. Procédé selon la revendication 8, comprenant en outre au moins l'une des opérations suivantes : modifier un visuel de l'interface utilisateur, générer un son par le biais d'un module audio et émettre une vibration, compte tenu du résultat de l'identification.

**12.** Procédé selon la revendication 8, comprenant en outre les opérations consistant à :

afficher l'interface utilisateur comprenant des informations de guidage permettant d'acquérir les premières données, et
émettre simultanément ou séquentiellement un son ou une vibration correspondant à des informations permettant de générer les premières données lorsque les informations permettant d'acquérir les premières données sont affichées.

**13.** Produit-programme informatique comprenant un support d'enregistrement non transitoire lisible par ordinateur stockant des instructions qui, lorsque le programme est exécuté par le processeur d'un appareil, amènent le processeur à effectuer les opérations comprenant les suivantes :

obtention de premières données liées à une première position d'un utilisateur mesurant sa pression artérielle (PA) grâce à l'appareil (1010),
identification d'une deuxième position de l'utilisateur mesurant sa PA, compte tenu de deuxièmes données acquises par le biais d'un module capteur (1020),
lesdites premières données et deuxièmes données renfermant respectivement en outre une inclinaison de l'appareil,
identification du fait qu'il existe ou non une différence entre les premières données et les deuxièmes données, y compris l'inclinaison de l'appareil ainsi que la hauteur à laquelle se trouve l'appareil électronique et/ou la distance entre l'appareil et l'utilisateur, est comprise dans un intervalle prédéfini, et
l'affichage d'une interface utilisateur, comprenant des informations de guidage permettant d'ajuster la deuxième position de façon qu'elle soit identique à la première position, compte tenu du résultat de l'identification (1030), ce qui comprend en outre l'affichage d'une première inclinaison de l'appareil électronique stockée en tant que premières données et d'une deuxième inclinaison de l'appareil électronique stockée en tant que deuxièmes données,
lesdites informations de guidage comprenant un premier objet graphique indiquant une position de référence et correspondant à la première position, et un deuxième objet graphique indiquant une position actuelle et correspondant à la deuxième position.

[Fig. 1]

[Fig. 2]

101

[Fig. 3]

300

[Fig. 4A]

[Fig. 4B]

[Fig. 5A]

[Fig. 5B]

[Fig. 5C]

**100**

10

INFO

**Blood Pressure**

530

♥ Blood pressure
setting reference inclination > > >

531

Hold electronic device and
change inclination thereof left
to right, then stop in
comfortable position.

Complete

540

[Fig. 6A]

**101**

30

610

♥ Blood pressure
Setting reference height

611

Slowly lower arm and
then raise your arm
over head, then stop
in comfortable position
at height adjacent
to heart.

[Fig. 6B]

**101**

[Fig. 7A]

**100** 10

[Fig. 7B]

[Fig. 7C]

[Fig. 8A]

**101**

810　　　　　　30

♥ Blood pressure
Measuring blood pressure >>>

Lower arm and then
slowly raise arm.
When you reach measurement
height, you are informed
through vibration

811

Current height　　Reference
height

815

817

813

[Fig. 8B]

**101**

820　　　　　　30

♥ Blood pressure
Measuring blood pressure >>>

Unfold arm outwardly from chest.
When you reach measurement
distance, you are informed
through vibration

Current
distance

Reference
height

823　　　　　　　821

825　　　　827

[Fig. 9]

**100**

[Fig. 10]

1000

START

1010
ACQUIRE FIRST DATA RELATED TO FIRST POSITION OF USER MEASURING BLOOD PRESSURE

1020
IDENTIFY SECOND POSITION OF USER THROUGH SECOND DATA ACQUIRED THROUGH SENSOR MODULE

1030
DISPLAY USER INTERFACE INCLUDING GUIDE INFORMATION FOR MAKING SECOND POSITION BECOME EQUAL TO FIRST POSITION, BASED ON IDENTIFICATION RESULT

END

[Fig. 11]

1100

```
           ┌─────────────┐
           │    START    │
           └──────┬──────┘
                  │              ⟋1110
        ┌─────────▼──────────────────┐
        │  START MEASURING BLOOD PRESSURE │
        └─────────┬──────────────────┘
                  │              ⟋1120
        ┌─────────▼──────────────────┐
        │ MEASURE HEIGHT OF ELECTRONIC DEVICE │
        │     AT TIME POINT OF MEASUREMENT    │
        └─────────┬──────────────────┘
                  │              ⟋1130
        ┌─────────▼──────────────────┐
        │ MEASURE DISTANCE BETWEEN USER  │
        │ AND ELECTRONIC DEVICE AT TIME  │
        │      POINT OF MEASUREMENT      │
        └─────────┬──────────────────┘
                  │              ⟋1140
        ┌─────────▼──────────────────┐
        │ MEASURE INCLINATION OF ELECTRONIC │
        │ DEVICE AT TIME POINT OF MEASUREMENT │
        └─────────┬──────────────────┘
                  │              ⟋1150
        ┌─────────▼──────────────────┐
        │    MEASURE BLOOD PRESSURE    │
        └─────────┬──────────────────┘
                  │              ⟋1160
        ┌─────────▼──────────────────┐
        │       STORE FIRST DATA       │
        └─────────┬──────────────────┘
                  │
           ┌──────▼──────┐
           │     END     │
           └─────────────┘
```

[Fig. 12]

```
                    ┌──────────┐
                    │  START   │
                    └────┬─────┘
                         │              ⌐1200
                         ▼
           ┌─────────────────────────────┐  ⌐1210
           │  START MEASURING BLOOD       │
           │  PRESSURE                    │
           └──────────────┬──────────────┘
                          │◄───────────────────────────────────┐
                          ▼                                     │
           ┌─────────────────────────────┐  ⌐1220              │
           │  MEASURE HEIGHT OF ELECTRONIC│                     │
           │  DEVICE AT TIME POINT OF     │         ⌐1240       │
           │  MEASUREMENT                 │  ┌──────────────────┴──────┐
           └──────────────┬──────────────┘  │  GENERATE GUIDE FOR     │
                          │                  │  HEIGHT OF ELECTRONIC   │
                          ▼                  │  DEVICE                 │
                    ╱ARE HEIGHT OF╲ ⌐1230    └─────────────▲───────────┘
                  ╱ ELECTRONIC DEVICE╲                     │
                 ╱ AT TIME POINT OF    ╲     NO            │
                ╱ MEASUREMENT AND PRE-   ╲────────────────►┘
                ╲ STORED HEIGHT OF       ╱
                 ╲ ELECTRONIC DEVICE IN ╱
                  ╲ ALLOWABLE RANGE?   ╱
                    ╲               ╱
                      │ YES
                      ▼◄──────────────────────────────────────┐
           ┌─────────────────────────────┐  ⌐1250            │
           │  MEASURE DISTANCE BETWEEN    │                   │
           │  USER AND ELECTRONIC DEVICE  │       ⌐1270       │
           │  AND INCLINATION AT TIME     │  ┌────────────────┴─────────┐
           │  POINT OF MEASUREMENT        │  │  GENERATE GUIDE FOR      │
           └──────────────┬──────────────┘  │  DISTANCE BETWEEN USER    │
                          │                 │  AND ELECTRONIC DEVICE    │
                          ▼                 │  AND INCLINATION OF       │
                  ╱ARE DISTANCE  ╲          │  ELECTRONIC DEVICE        │
                ╱ BETWEEN USER AND ╲ ⌐1260  └────────────▲─────────────┘
               ╱ ELECTRONIC DEVICE AT╲                   │
              ╱ TIME POINT OF          ╲                 │
             ╱ MEASUREMENT AND PRE-      ╲   NO           │
            ╱ STORED DISTANCE BETWEEN USER╲───────────────┘
            ╲ AND ELECTRONIC DEVICE, AND  ╱
             ╲ INCLINATION OF ELECTRONIC ╱
              ╲ DEVICE AT TIME POINT    ╱
               ╲ OF MEASUREMENT AND    ╱
                ╲ PRE-STORED          ╱
                 ╲ INCLINATION OF    ╱
                  ╲ ELECTRONIC      ╱
                   ╲ DEVICE IN     ╱
                    ╲ ALLOWABLE   ╱
                     ╲ RANGE?    ╱
                        │ YES
                        ▼          ⌐1280
           ┌─────────────────────────────┐
           │  START MEASURING BLOOD       │
           │  PRESSURE THROUGH SENSOR     │
           └──────────────┬──────────────┘
                          ▼
                    ┌──────────┐
                    │   END    │
                    └──────────┘
```

[Fig. 13]

1300

```
              ┌─────────┐
              │  START  │
              └────┬────┘
                   ▼
          ┌──────────────────────────┐  1310
          │ START MEASURING BLOOD    │
          │ PRESSURE                 │
          └──────────┬───────────────┘
                     ▼                                    1315
          ┌──────────────────────────┐
          │ MEASURE HEIGHT OF        │
          │ ELECTRONIC DEVICE        │              ┌──────────────────────┐ 1325
          │ AT TIME POINT OF         │              │ GENERATE GUIDE TO    │
          │ MEASUREMENT              │              │ HEIGHT OF ELECTRONIC │
          └──────────┬───────────────┘              │ DEVICE               │
                     ▼                               └──────────┬───────────┘
              ◇ ARE HEIGHT                                      ▲
              OF ELECTRONIC DEVICE  1320                        │
              AT TIME POINT OF MEASUREMENT    NO                │
              AND PRE-STORED HEIGHT OF ──────────────────────────
              ELECTRONIC DEVICE IN
              ALLOWABLE RANGE?
                     │ YES
                     ▼                                    1330
          ┌──────────────────────────┐
          │ MEASURE DISTANCE BETWEEN │
          │ USER AND ELECTRONIC      │              ┌──────────────────────┐ 1340
          │ DEVICE AT TIME POINT OF  │              │ GENERATE GUIDE FOR   │
          │ MEASUREMENT              │              │ DISTANCE BETWEEN     │
          └──────────┬───────────────┘              │ ELECTRONIC DEVICE    │
                     ▼                               │ AND USER             │
              ◇ ARE DISTANCE 1335                    └──────────┬───────────┘
              BETWEEN USER AND                                  ▲
              ELECTRONIC DEVICE AT TIME POINT                   │
              OF MEASUREMENT AND PRE-STORED    NO               │
              DISTANCE BETWEEN USER AND ────────────────────────
              ELECTRONIC DEVICE IN
              ALLOWABLE RANGE?
                     │ YES
                     ▼                                    1345
          ┌──────────────────────────┐
          │ MEASURE INCLINATION OF   │              ┌──────────────────────┐ 1355
          │ ELECTRONIC DEVICE AT     │              │ GENERATE GUIDE FOR   │
          │ TIME POINT OF MEASUREMENT│              │ INCLINATION OF       │
          └──────────┬───────────────┘              │ ELECTRONIC DEVICE    │
                     ▼                               └──────────┬───────────┘
              ◇ ARE INCLINATION 1350                            ▲
              OF ELECTRONIC DEVICE AT                           │
              TIME POINT OF MEASUREMENT AND    NO               │
              PRE-STORED INCLINATION OF ────────────────────────
              ELECTRONIC DEVICE IN ALLOWABLE
              RANGE?
                     │ YES                  1360
                     ▼
          ┌──────────────────────────┐
          │ START MEASURING BLOOD    │
          │ PRESSURE THROUGH SENSOR  │
          └──────────┬───────────────┘
                     ▼
              ┌─────────┐
              │   END   │
              └─────────┘
```

[Fig. 14A]

[Fig. 14B]

[Fig. 15A]

[Fig. 15B]

[Fig. 16A]

[Fig. 16B]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110172547 A1, Tomohiro Izumi

- WO 2014094245 A1, Yi Liu